(19) Europäisches Patentamt
European Patent Office
Office européen des brevets

(11) EP 4 151 614 A1

(12) EUROPEAN PATENT APPLICATION
published in accordance with Art. 153(4) EPC

(43) Date of publication:
22.03.2023 Bulletin 2023/12

(21) Application number: 21804193.7

(22) Date of filing: 11.05.2021

(51) International Patent Classification (IPC):
*C07C 63/72* (2006.01) *C07C 211/52* (2006.01)
*C08G 73/10* (2006.01) *C08G 73/18* (2006.01)

(52) Cooperative Patent Classification (CPC):
**C07C 63/72; C07C 211/52; C07C 211/53; C07D 251/30; C08G 69/32; C08G 73/10; C08G 73/18; H05K 1/03**

(86) International application number:
**PCT/JP2021/017952**

(87) International publication number:
**WO 2021/230255 (18.11.2021 Gazette 2021/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.05.2020 JP 2020083305**

(71) Applicants:
- **Daikin Industries, Ltd.**
**Osaka-shi, Osaka 530-0001 (JP)**
- **NATIONAL UNIVERSITY CORPORATION, IWATE UNIVERSITY**
**Morioka-shi, Iwate 020-8550 (JP)**

(72) Inventors:
- **OISHI, Yoshiyuki**
**Morioka-shi, Iwate 020-8550 (JP)**
- **NOGUCHI, Tsuyoshi**
**Osaka-shi, Osaka 530-8323 (JP)**

(74) Representative: **Hoffmann Eitle**
**Patent- und Rechtsanwälte PartmbB**
**Arabellastraße 30**
**81925 München (DE)**

(54) **FLUORINATED AMIDE COMPOUND, COMPOUND CONTAINING FLUORINATED NITROGEN-CONTAINING HETEROCYCLIC RING, AND FLUORINATED COMPOUND**

(57) Provided is a fluorinated amide compound having a specific repeating unit and a fluorinated nitrogen-containing heterocyclic ring-containing compound having a specific repeating unit.

EP 4 151 614 A1

## Description

TECHNICAL FIELD

The present disclosure relates to a fluorinated amide compound, a fluorinated nitrogen-containing heterocyclic ring-containing compound, and a fluorinated compound.

BACKGROUND ART

Non Patent Document 1 and Patent Document 1 describe that polyimide and polybenzimidazole have the highest level of heat resistance, strength, and chemical stability as polymer materials, and used in various fields as heat-resistant fibers, molded articles, coating varnishes, and the like.

RELATED ART

NON PATENT DOCUMENT

Non Patent Document 1: Japan Polyimide Research Group, "Latest Polyimides -Basics and Applications-", NTS Co., Ltd., published January 28, 2002, pp. 292 to 326.

PATENT DOCUMENT

Patent Document 1: Japanese Patent Laid-Open No. 9-208699

SUMMARY OF THE INVENTION

PROBLEM TO BE SOLVED BY THE INVENTION

It is an object of the present disclosure to provide a fluorinated amide compound having a low dielectric constant and a low dielectric loss.
Further, it is an object of the present disclosure to provide a fluorinated nitrogen-containing heterocyclic ring-containing compound having a low dielectric constant and a low dielectric loss.
Further, it is an object of the present disclosure to provide a novel fluorinated compound.

MEANS FOR SOLVING THE PROBLEM

According to the present disclosure, there is provided a fluorinated amide compound including a repeating unit represented by Formula (1).

Formula (1):

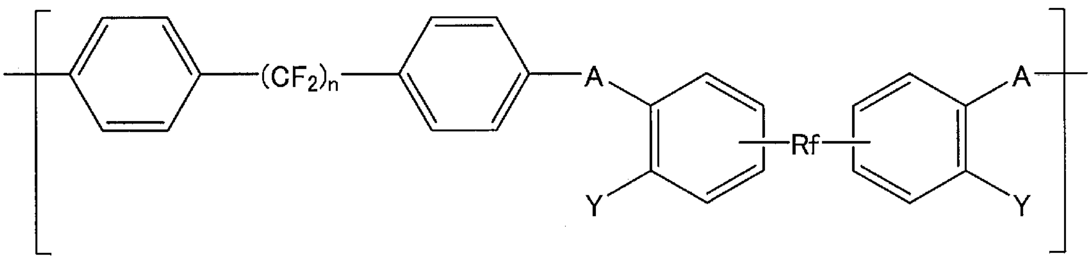

(In Formula (1), n represents an integer of 4 to 8, Rf represents a single bond, $-SO_2-$, -O-, -CO-, a divalent non-fluorinated organic group, or a divalent fluorinated organic group, A represents an amide bond, each Y independently represents $-COR^1$ or $-NHR^2$, each $R^1$ independently represents OH, an optionally-substituted linear or branched chain alkoxy group, an optionally-substituted aromatic oxy group, or a halogen atom, and each $R^2$ independently represents H or a monovalent organic group.)
The average degree of polymerization of the repeating unit represented by Formula (1) is preferably 2 to 100.
The repeating unit represented by Formula (1) is preferably a repeating unit represented by Formula (1a).

Formula (1a):

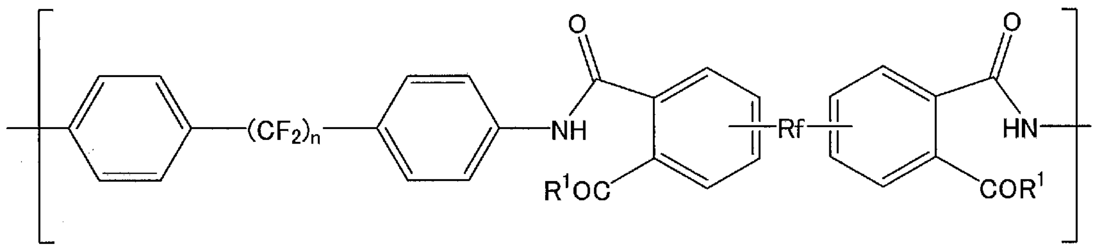

(In Formula (1a), n represents an integer of 4 to 8, Rf represents a single bond, $-SO_2-$, -O-, -CO-, a divalent non-fluorinated organic group, or a divalent fluorinated organic group, and each $R^1$ independently represents OH, an optionally-substituted linear or branched chain alkoxy group, an optionally-substituted aromatic oxy group, or a halogen atom.)

The repeating unit represented by Formula (1) is preferably a repeating unit represented by Formula (1b).

Formula (1b):

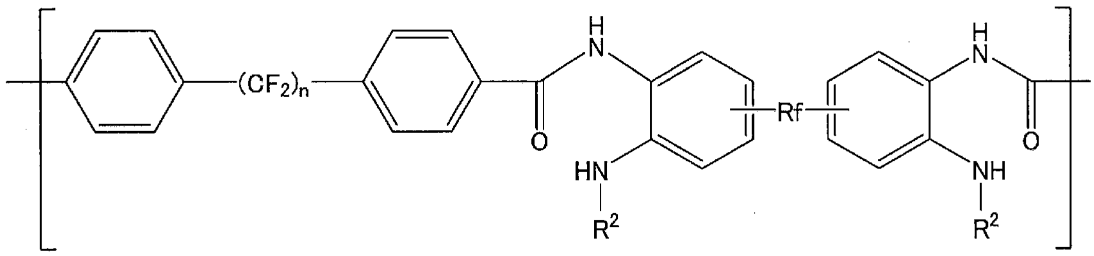

(In Formula (1b), n represents an integer of 4 to 8, Rf represents a single bond, $-SO_2-$, -O-, -CO-, a divalent non-fluorinated organic group, or a divalent fluorinated organic group, and each $R^2$ independently represents H or a monovalent organic group.)

Further, according to the present disclosure, there is provided a fluorinated nitrogen-containing heterocyclic ring-containing compound including a repeating unit represented by Formula (3).

Formula (3):

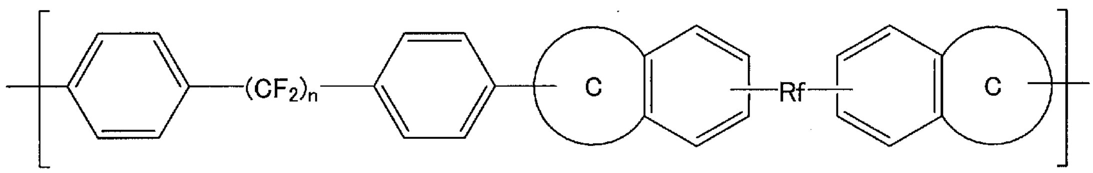

(In Formula (3), n represents an integer of 4 to 8, Rf represents a single bond, $-SO_2-$, -O-, -CO-, a divalent non-fluorinated organic group, or a divalent fluorinated organic group, and a ring C represents an optionally-substituted imide ring or benzimidazole ring.)

The average degree of polymerization of the repeating unit represented by Formula (3) is preferably 2 to 100.

The repeating unit represented by Formula (3) is preferably a repeating unit represented by Formula (3a).

Formula (3a):

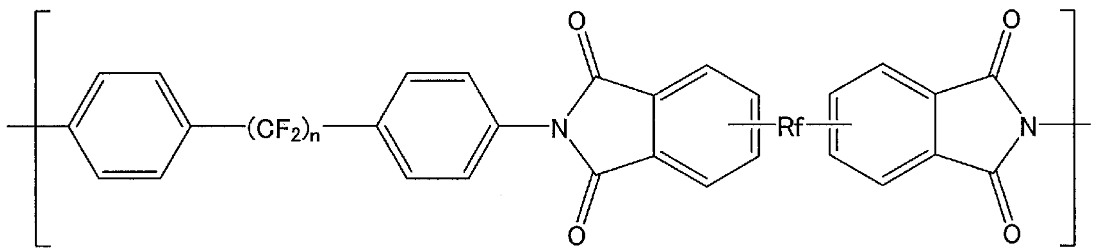

(In Formula (3a), n represents an integer of 4 to 8, and Rf represents a single bond, $-SO_2-$, -O-, -CO-, a divalent non-fluorinated organic group, or a divalent fluorinated organic group.)

The repeating unit represented by Formula (3) is preferably a repeating unit represented by Formula (3b).

Formula (3b):

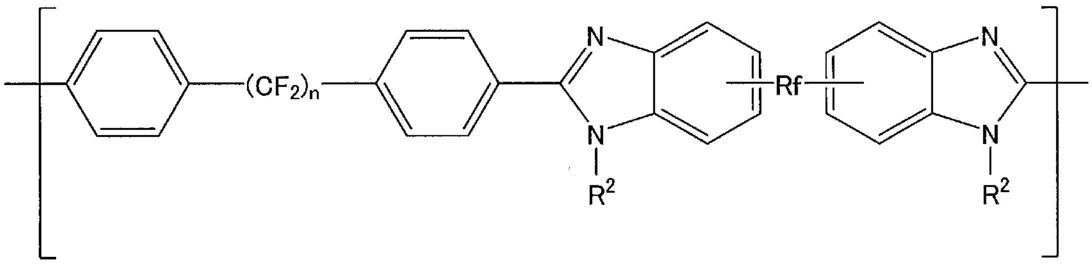

(In Formula (3b), n represents an integer of 4 to 8, Rf represents a single bond, $-SO_2-$, -O-, -CO-, a divalent non-fluorinated organic group, or a divalent fluorinated organic group, and each $R^2$ independently represents H or a monovalent organic group.)

Further, according to the present disclosure, there is provided a low dielectric material containing the above-mentioned fluorinated amide compound or the above-mentioned fluorinated nitrogen-containing heterocyclic ring-containing compound.

Further, according to the present disclosure, there is provided a printed circuit board containing the above-mentioned fluorinated amide compound or the above-mentioned fluorinated nitrogen-containing heterocyclic ring-containing compound.

Further, according to the present disclosure, there is provided a fluorinated compound (4a-1) represented by Formula (4a-1).

Formula (4a-1):

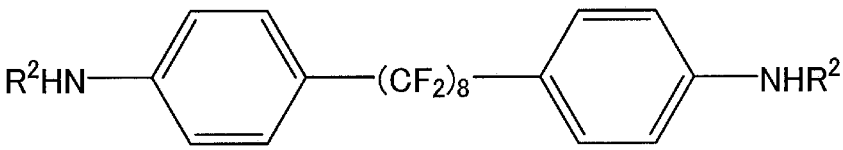

(In Formula (4a-1), each $R^2$ independently represents H or a monovalent organic group.)

Further, according to the present disclosure, there is provided a fluorinated compound (4B) represented by Formula (4B).

Formula (4B):

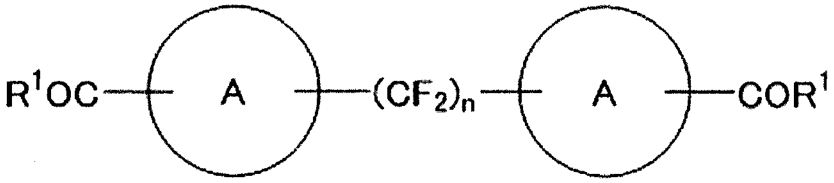

(In Formula (4B), n represents an integer of 4 to 8, ring A represents a hydrocarbon ring, and each $R^1$ independently represents OH, an optionally-substituted linear or branched chain alkoxy group, an optionally-substituted aromatic oxy group, or a halogen atom.)

EFFECTS OF INVENTION

According to the present disclosure, there can be provided a fluorinated amide compound having a low dielectric constant and a low dielectric loss.

Further, according to the present disclosure, there can be provided a fluorinated nitrogen-containing heterocyclic ring-containing compound having a low dielectric constant and a low dielectric loss.

Further, according to the present disclosure, there can be provided a novel fluorinated compound.

DESCRIPTION OF EMBODIMENTS

Hereinafter, specific embodiments of the present disclosure will be described in detail, but the present disclosure is not limited to the following embodiments.

<Fluorinated amide compound>

The fluorinated amide compound of the present disclosure has a repeating unit represented by Formula (1).

Formula (1):

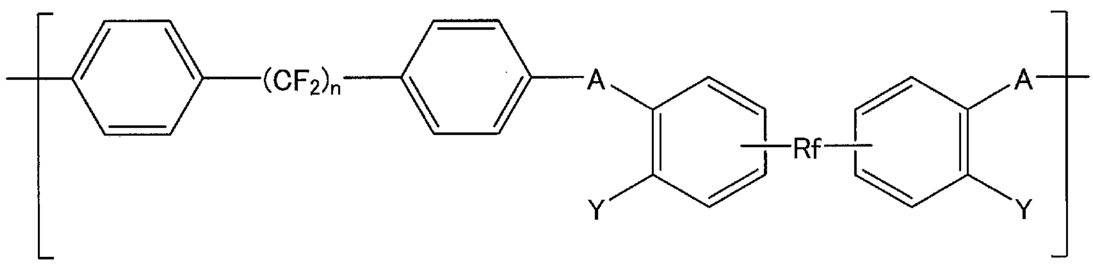

(In Formula (1), n represents an integer of 4 to 8, Rf represents a single bond, $-SO_2-$, -O-, -CO-, a divalent non-fluorinated organic group, or a divalent fluorinated organic group, A represents an amide bond, each Y independently represents $-COR^1$ or $-NHR^2$, each $R^1$ independently represents OH, an optionally-substituted linear or branched chain alkoxy group, an optionally-substituted aromatic oxy group, or a halogen atom, and each $R^2$ independently represents H or a monovalent organic group.)

n represents an integer of 4 to 8. From the perspective that the dielectric constant and dielectric loss of the fluorinated amide compound can be made much lower, n is preferably an integer of 6 to 8, more preferably 6 or 8, and further preferably 8.

Rf represents a single bond, $-SO_2-$, -O-, -CO-, a divalent non-fluorinated organic group, or a divalent fluorinated organic group. From the perspective that the dielectric constant and dielectric loss of the fluorinated amide compound can be made much lower, Rf is preferably a single bond, -O-, -CO-, or a divalent fluorinated organic group, more preferably -O- or a divalent fluorinated organic group, and further preferably a divalent fluorinated organic group.

The non-fluorinated organic group is a divalent organic group that does not have a fluorine atom. The non-fluorinated organic group is preferably a linear or branched chain non-fluorinated alkylene group or a divalent non-fluorinated aryl group.

The fluorinated organic group is a divalent organic group having one or more fluorine atoms. From the perspective that the dielectric constant and dielectric loss of the fluorinated amide compound can be made much lower, Rf

is preferably a linear or branched chain fluorinated alkylene group.

From the perspective that the dielectric constant and dielectric loss of the fluorinated amide compound can be made much lower, the fluorinated alkylene group is preferably a perfluoroalkylene group.

From the perspective that the dielectric constant and dielectric loss of the fluorinated amide compound can be made much lower, the number of carbons of the fluorinated alkylene group is preferably 1 to 15, and more preferably 2 or more, and more preferably 10 or less, and further preferably 5 or less.

From the perspective that the dielectric constant and dielectric loss of the fluorinated amide compound can be made much lower, the fluorinated alkylene group is preferably $-C(CF_3)_2-$.

Each $R^1$ is independently OH, an optionally-substituted linear or branched chain alkoxy group, an optionally-substituted aromatic oxy group, or a halogen atom.

The number of carbon atoms of the alkoxy group as $R^1$ is preferably 1 to 12, and more preferably 1 to 6.

The optional substituent of the alkoxy group and the aromatic oxy group as $R^1$ is preferably an alkoxy group, an alkyl group, a fluorinated alkyl group, a halo group (halogen atom), a nitro group, a cyano group, or an ester group, and more preferably is an alkoxy group.

Examples of the aromatic oxy group as $R^1$ include a phenoxy group that does not have a substituent, an optionally-substituted triazinyloxy group, and the like.

It is preferable that each $R^1$ is independently OH, a phenoxy group that does not have a substituent, a methoxy group, an ethoxy group, a chlorine atom, or the following.

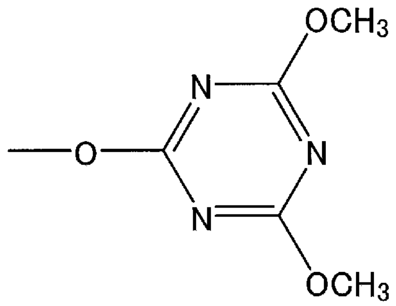

Each $R^2$ is independently H or a monovalent organic group. The monovalent organic group is a monovalent group containing a carbon atom or a group formed by removing one hydrogen atom from an organic compound. Examples of the monovalent organic group include an optionally-substituted aliphatic hydrocarbon group and an optionally-substituted aromatic group.

Specific examples of the monovalent organic group include a lower alkyl group having 1 to 10 carbon atoms, and particularly 1 to 6 carbon atoms, such as $-CH_3$, $-C_2H_5$, and $-C_3H_7$; a fluorine atom-containing lower alkyl group having 1 to 10 carbon atoms, and particularly 1 to 6 carbon atoms, such as $-CF_3$, $-C_2F_5$, $-CH_2F$, $-CH_2CF_3$, and $-CH_2C_2F_5$; a phenyl group (without a substituent); a benzyl group (without a substituent); a phenyl group or a benzyl group in which 1 to 5 hydrogen atoms are substituted with a fluorine atom, such as $-C_6F_5$ and $-CH_2C_6F_5$; and a phenyl group or a benzyl group in which 1 to 5 hydrogen atoms are substituted with a $-CF_3$, such as $-C_6H_{5-k}(CF_3)_k$, $-CH_2C_6H_{5-k}(CF_3)_k$ (k is an integer of 1 to 5) .

It is preferable that each $R^2$ is independently H or an optionally-substituted aromatic group, more preferably H or an optionally-substituted phenyl group, and further preferably H, a phenyl group not having a substituent, or a phenyl group substituted with a fluorine atom-containing alkyl group having 1 to 10 carbon atoms.

From the perspective that the dielectric constant and dielectric loss of the fluorinated amide compound can be made much lower, $R^2$ is preferably a phenyl group not having a substituent or a phenyl group substituted with a fluorine atom-containing alkyl group having 1 to 10 carbon atoms.

The repeating unit represented by Formula (1) is preferably a repeating unit represented by Formula (1a).

Formula (1a):

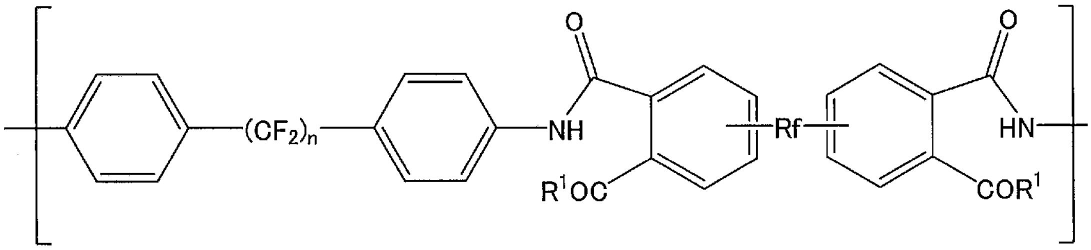

(In Formula (1a), n, Rf, and $R^1$ are the same as in Formula (1).)

Examples of the fluorinated amide compound containing the repeating unit represented by Formula (1a) include a compound represented by the following formula.

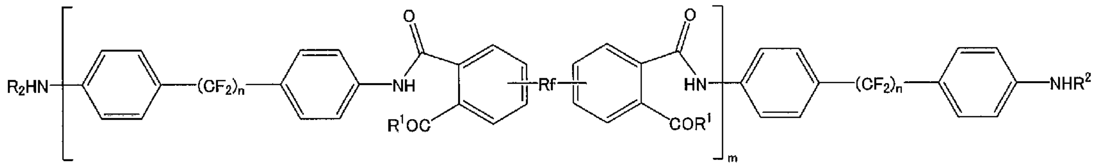

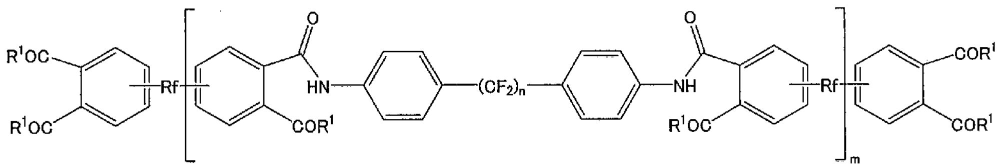

(In the formula, n, Rf, $R^1$, and $R^2$ are the same as in Formula (1). m represents the average degree of polymerization of the repeating unit represented by Formula (1a).)

By containing a repeating unit represented by Formula (1a), the fluorinated amide compound of the present disclosure has a much lower dielectric constant and dielectric loss. Further, for the repeating unit represented by Formula (1), the fluorinated amide compound containing the repeating unit represented by Formula (1a) can be suitably used as a precursor of the fluorinated nitrogen-containing heterocyclic ring-containing compound (imide compound) having the repeating unit represented by Formula (3a).

In Formula (1a), n, Rf, $R^1$, and $R^2$ are the same as in Formula (1), and by having the same suitable structure as Formula (1), the dielectric constant and the dielectric loss of the fluorinated amide compound can be much lower.

Further, the repeating unit represented by Formula (1) is preferably a repeating unit represented by Formula (1b).

Formula (1b):

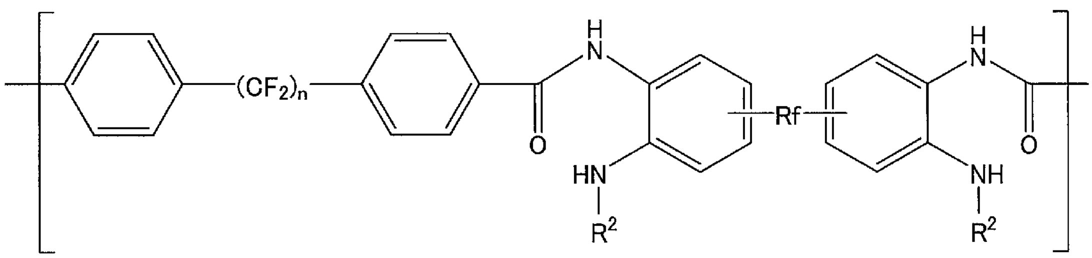

(In Formula (1b), n, Rf, and $R^2$ are the same as in Formula (1).)

Examples of the fluorinated amide compound containing a repeating unit represented by Formula (1b) include a compound represented by any of the following formulas.

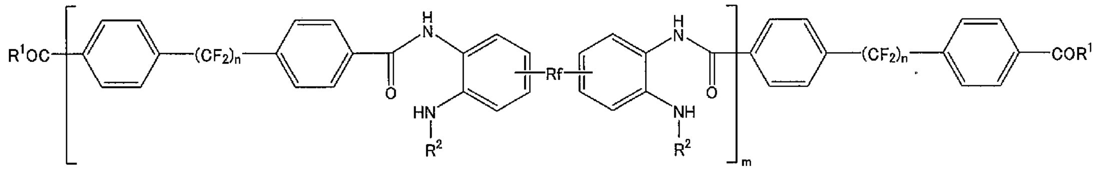

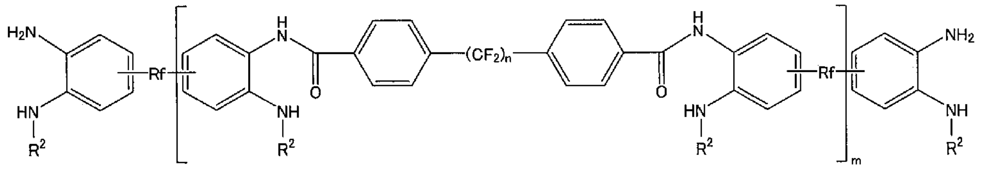

(In the formula, n, Rf, $R^1$, and $R^2$ are the same as in Formula (1). m represents the average degree of polymerization of the repeating unit represented by Formula (1b).)

By containing a repeating unit represented by Formula (1b), the fluorinated amide compound of the present disclosure has a much lower dielectric constant and dielectric loss. Further, for the repeating unit represented by Formula (1), the fluorinated amide compound containing the repeating unit represented by Formula (1b) can be suitably used as a precursor of the fluorinated nitrogen-containing heterocyclic ring-containing compound (benzimidazole compound) having the repeating unit represented by Formula (3b).

In Formula (1b), n, Rf, $R^1$, and $R^2$ are the same as in Formula (1), and by having the same suitable structure as Formula (1), the dielectric constant and the dielectric loss of the fluorinated amide compound can be much lower.

In the fluorinated amide compound of the present disclosure, the average degree of polymerization of the repeating unit represented by Formula (1) is preferably 200 or less, more preferably 150 or less, and further preferably 100 or less, and may be 2 or more, or may be 3 or more. The average degree of polymerization is determined based on a calculation from the number average molecular weight of the fluorinated amide compound of the present disclosure.

The number average molecular weight (Mn) of the fluorinated amide compound of the present disclosure is, in terms of standard polystyrene by gel permeation chromatography, preferably 10,000 or more, and more preferably 20,000 or more, and preferably 1,000,000 or less, and more preferably 500,000 or less.

The molecular weight distribution (Mw/Mn) of the fluorinated amide compound of the present disclosure is, in terms of standard polystyrene by gel permeation chromatography, preferably 1.2 or more, and more preferably 2 or more, and preferably 5 or less, and more preferably 4 or less.

The logarithmic viscosity $\eta_{inh}$ of the fluorinated amide compound of the present disclosure is preferably 0.3 dL/g or more, and more preferably 0.5 dL/g or more. The logarithmic viscosity $\eta_{inh}$ can be calculated by the following formula by dissolving the fluorinated amide compound in N-methyl-2-pyrrolidone (NMP) or the like as a solvent to prepare a solution having a solution concentration of 0.5 g/dL and measuring the viscosity (solution viscosity) of the prepared solution at 30°C.

$$\text{Logarithmic viscosity } \eta_{inh} = \ln(\text{solution viscosity} / \text{solvent viscosity}) / \text{solution concentration}$$

The fluorinated amide compound of the present disclosure can be suitably used as a precursor of the fluorinated nitrogen-containing heterocyclic ring-containing compound having a repeating unit represented by Formula (3) described below.

<Fluorinated nitrogen-containing heterocyclic ring-containing compound>

The fluorinated nitrogen-containing heterocyclic ring-containing compound of the present disclosure includes a repeating unit represented by Formula (3).

Formula (3):

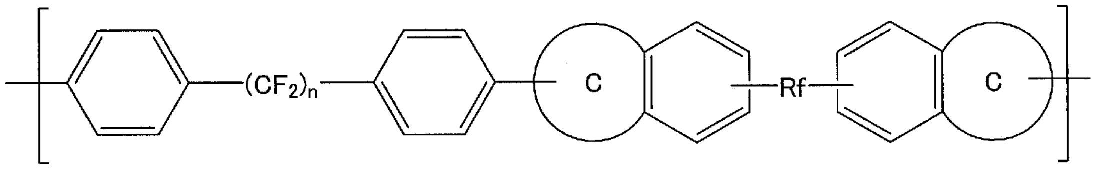

(In Formula (3), n represents an integer of 4 to 8, Rf represents a single bond, $-SO_2-$, -O-, -CO-, a divalent non-fluorinated organic group, or a divalent fluorinated organic group, and a ring C represents an optionally-substituted imide ring or benzimidazole ring.)

n and Rf are the same as in Formula (1). By having the same suitable structures for n and Rf as in Formula (1), the dielectric constant and the dielectric loss of the fluorinated nitrogen-containing heterocyclic ring-containing compound can be much lower.

The ring C is an optionally-substituted imide ring or benzimidazole ring. It is preferable that the imide ring does not have a substituent. The benzimidazole ring is optionally substituted. Examples of the substituent that benzimidazole ring can have include a monovalent organic group, and the groups described for $R^2$ of Formula (1) are preferable.

The repeating unit represented by Formula (3) is preferably a repeating unit represented by Formula (3a).

Formula (3a):

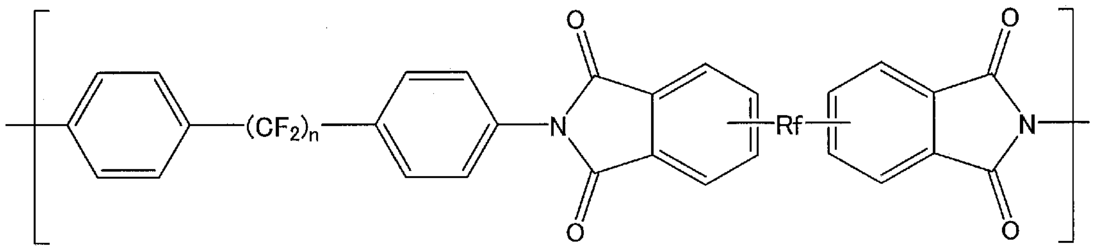

(In Formula (3a), n and Rf are the same as in Formula (1).)

Examples of the fluorinated nitrogen-containing heterocyclic ring-containing compound containing the repeating unit represented by Formula (3a) include a compound represented by the following formula.

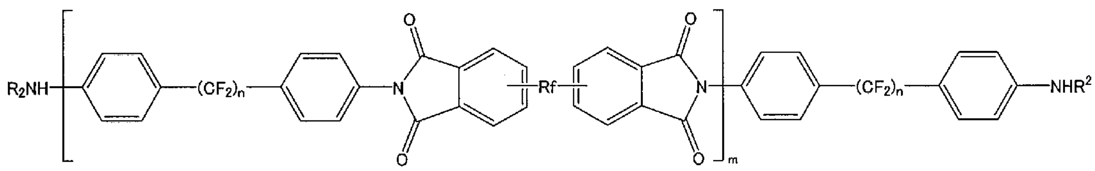

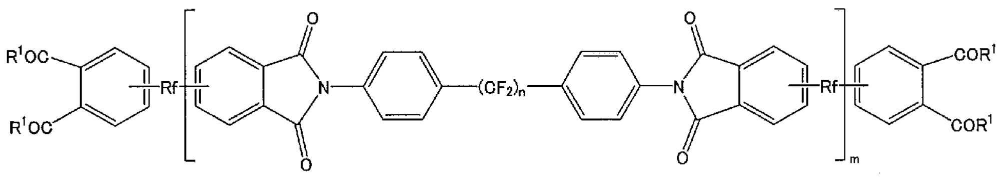

(In the formula, n, Rf and $R^2$ are the same as in Formula (1). m represents the average degree of polymerization of the repeating unit represented by Formula (3a).)

By containing a repeating unit represented by Formula (3a), the fluorinated nitrogen-containing heterocyclic ring-containing compound of the present disclosure has a much lower dielectric constant and dielectric loss.

In Formula (3a), n and Rf are the same as in Formula (1), and by having the same suitable structure as Formula (1), the dielectric constant and the dielectric loss of the fluorinated nitrogen-containing heterocyclic ring-containing compound can be much lower.

Further, the repeating unit represented by Formula (3) is preferably a repeating unit represented by Formula (3b).

Formula (3b):

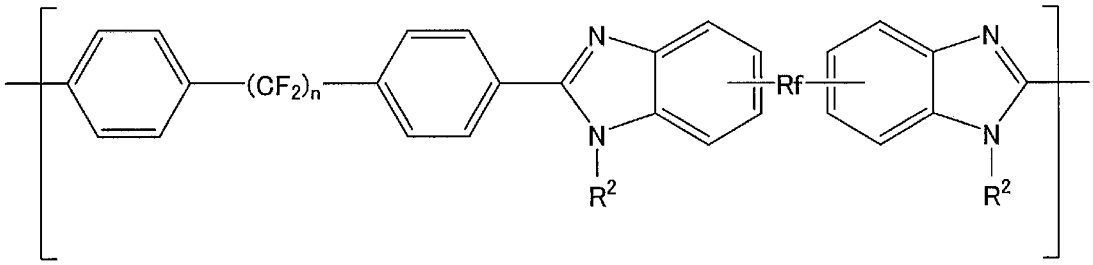

(In Formula (3b), n, Rf and $R^2$ are the same as in Formula (1).)

Examples of the fluorinated nitrogen-containing heterocyclic ring-containing compound containing the repeating unit represented by Formula (3b) include a compound represented by the following formula.

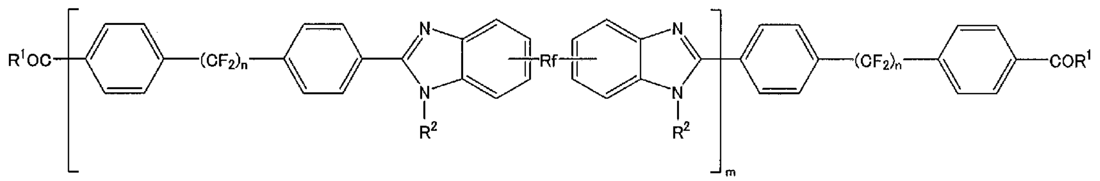

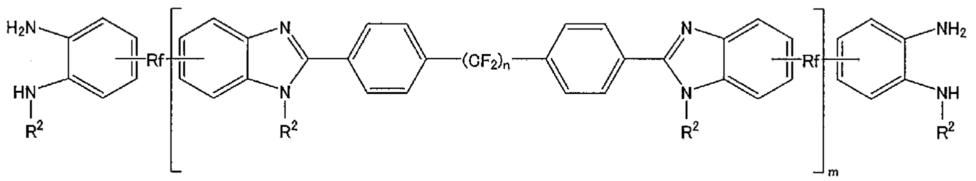

(In the formula, n, Rf, $R^1$, $R^2$, and Y are the same as in Formula (1) . m represents the average degree of polymerization of the repeating unit represented by Formula (3b).)

By containing a repeating unit represented by Formula (3b), the fluorinated nitrogen-containing heterocyclic ring-containing compound of the present disclosure has a much lower dielectric constant and dielectric loss.

In Formula (3b), n, Rf and $R^2$ are the same as in Formula (1), and by having the same suitable structure as Formula (1), the dielectric constant and the dielectric loss of the fluorinated nitrogen-containing heterocyclic ring-containing compound can be much lower.

From the perspective that the dielectric constant and dielectric loss of the fluorinated nitrogen-containing heterocyclic ring-containing compound can be made much lower, the repeating unit represented by Formula (3b) is preferably a repeating unit represented by Formula (3b-1) or Formula (3b-2).

Formula (3b-1)

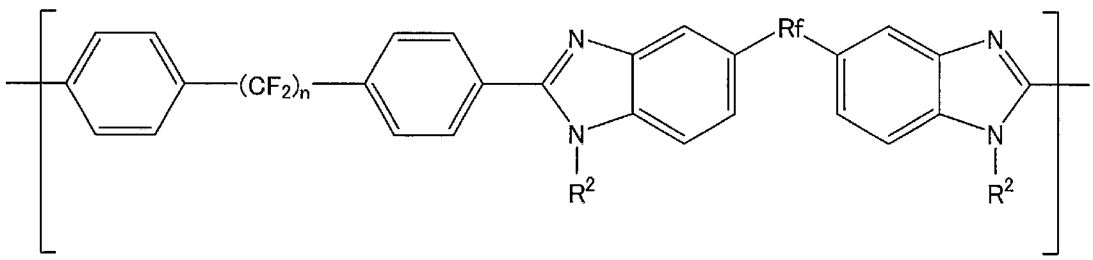

(In Formula (3b-1), n, Rf, and $R^2$ are the same as in Formula (1).)

Formula (3b-2)

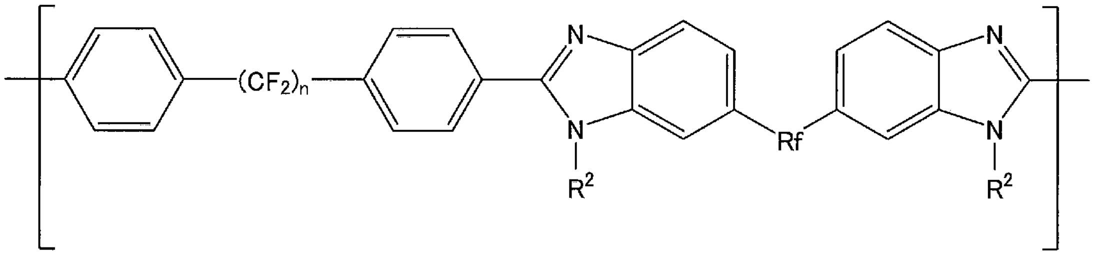

(In Formula (3b-2), n, Rf, and $R^2$ are the same as in Formula (1).)

The glass transition temperature of the fluorinated nitrogen-containing heterocyclic ring-containing compound of the present disclosure is preferably 50 to 400°C, more preferably 100 to 350°C, and further preferably 150 to 260°C. The glass transition temperature is a value measured by thermomechanical analysis (TMA), differential scanning calorimetry (DSC), or dynamic viscoelasticity measurement (DMA).

In the fluorinated nitrogen-containing heterocyclic ring-containing compound of the present disclosure, the average degree of polymerization of the repeating unit represented by Formula (3) is preferably 200 or less, more preferably 100 or less, and may be 2 or more, or may be 3 or more. The average degree of polymerization is determined based on a calculation from the number average molecular weight of the fluorinated nitrogen-containing heterocyclic ring-containing compound of the present disclosure.

From the perspective that the dielectric constant and dielectric loss of the fluorinated nitrogen-containing heterocyclic ring-containing compound can be made much lower, the fluorinated nitrogen-containing heterocyclic ring-containing compound is preferably a polymer having a comparatively large average degree of polymerization, for example, a polymer having an average degree of polymerization of more than 100.

The number average molecular weight (Mn) of the fluorinated nitrogen-containing heterocyclic ring-containing compound of the present disclosure is, in terms of standard polystyrene by gel permeation chromatography, preferably 10,000 or more, and more preferably 20,000 or more, and preferably 1,000,000 or less, and more preferably 50,000 or less.

The molecular weight distribution (Mw/Mn) of the fluorinated nitrogen-containing heterocyclic ring-containing compound of the present disclosure is, in terms of standard polystyrene by gel permeation chromatography, preferably 1.2 or more, and more preferably 2 or more, and preferably 5 or less, and more preferably 4 or less.

The logarithmic viscosity $\eta_{inh}$ of the fluorinated nitrogen-containing heterocyclic ring-containing compound of the present disclosure is preferably 0.3 dL/g or more, and more preferably 0.5 dL/g or more. The logarithmic viscosity $\eta_{inh}$ can be calculated by the following formula by dissolving the fluorinated nitrogen-containing heterocyclic ring-containing compound in N-methyl-2-pyrrolidone (NMP) or the like as a solvent to prepare a solution having a solution concentration of 0.5 g/dL and measuring the viscosity (solution viscosity) of the prepared solution at 30°C.

$$\text{Logarithmic viscosity } \eta_{inh} = \ln(\text{solution viscosity} / \text{solvent viscosity}) / \text{solution concentration}$$

<Method for producing fluorinated amide compound>

The fluorinated amide compound of the present disclosure can be suitably produced by producing a fluorinated compound (4) represented by Formula (4) based on the production method described later, and then polymerizing the obtained fluorinated compound (4) with a compound (5) represented by Formula (5).

Formula (4):

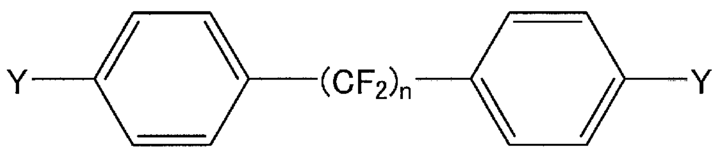

(In Formula (4), n and Y are the same as in Formula (1).)

Formula (5):

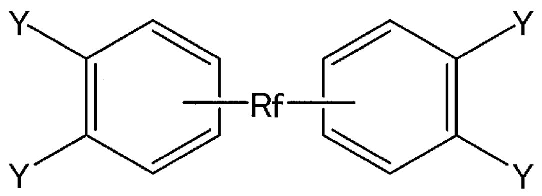

(In Formula (5), Rf and Y are the same as in Formula (1). However, when Y in Formula (4) is -$COR^1$, Y in Formula (5) is -$NHR^2$. Further, when Y in Formula (4) is -$NHR^2$, Y in Formula (5) is -$COR^1$. Further, when Y in Formula (5) is -$COR^1$, two adjacent Y may be bonded to each other via an acid anhydride bond (-CO-O-CO-) to form a ring together with the two carbon atoms that the two Y's are bonded to. $R^1$ and $R^2$ are the same as in Formula (1).)

As the fluorinated compound (4) and the compound (5), the fluorinated compound (4) is preferably a fluorinated compound (4a) represented by Formula (4a) and the compound (5) is preferably a compound (5a) represented by Formula (5a), or the fluorinated compound (4) is preferably a fluorinated compound (4b) represented by Formula (4b) and the compound (5) is preferably a compound (5b) represented by Formula (5b). The fluorinated amide compound having a repeating unit represented by Formula (1a) can be obtained by polymerizing the fluorinated compound (4a) and the compound (5a). Further, the fluorinated amide compound having a repeating unit represented by Formula (1b) can be obtained by polymerizing the fluorinated compound (4b) and the compound (5b).

Formula (4a):

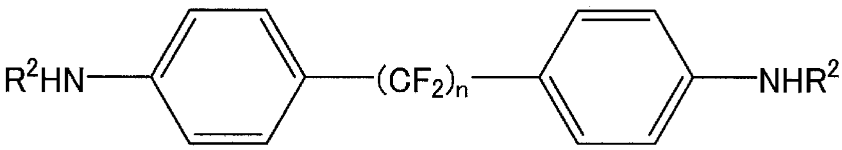

(In Formula (4a), n and $R^2$ are the same as in Formula (1).)

Formula (5a):

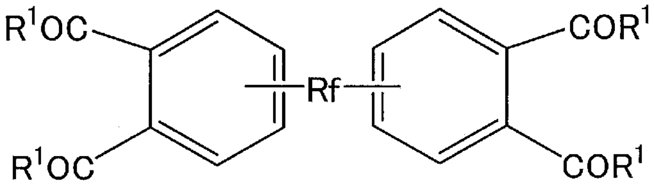

(In Formula (5a), Rf and $R^1$ are the same as in Formula (1).)

Formula (4b):

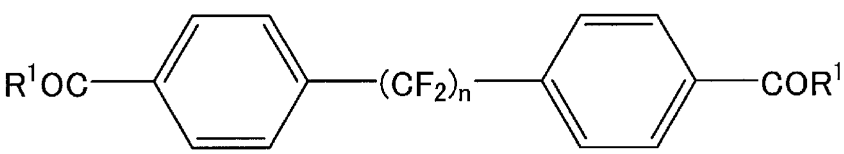

(In Formula (4b), n and $R^1$ are the same as in Formula (1).)

Formula (5b):

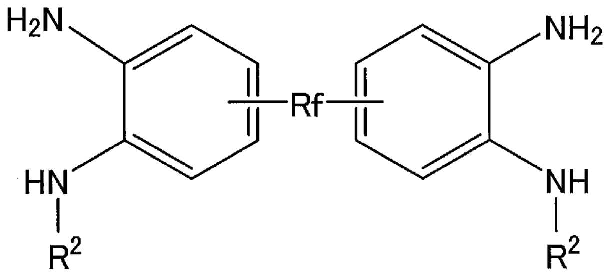

(In Formula (5b), Rf and $R^2$ are the same as in Formula (1).)

From the perspective that the dielectric constant and dielectric loss of the obtained fluorinated amide compound and fluorinated nitrogen-containing heterocyclic ring-containing compound can be made much lower, the fluorinated compound (4a) is preferably a fluorinated compound (4a-1) represented by Formula (4a-1).

Formula (4a-1):

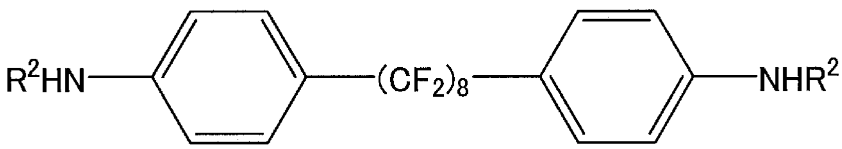

(In Formula (4a-1), $R^2$ is the same as in Formula (1).)

The polymerization of the fluorinated compound (4) and the compound (5) can be carried out in a solvent. It is desirable that the solvent does not substantially react with the fluorinated compound (4) and the compound (5), has the property of dissolving the fluorinated compound (4) and the compound (5) well, and is a good solvent for the compound obtained by polymerizing the fluorinated compound (4) and the compound (5). Such a solvent is not limited, and examples thereof include dimethyl sulfoxide (DMSO), N,N-dimethylformamide (DMF), N,N-dimethylacetamide (DMAc), N-methyl-2-pyrrolidone (NMP), 1,3-dimethylimidazolidone (DMI), sulfolane, tetrahydrofuran (THF), and acetone. Of these, N-methyl-2-pyrrolidone (NMP) and 1,3-dimethylimidazolidone (DMI) are preferable. The amount of these solvents used is, based on 0.1 mol of the fluorinated compound (4) or the compound (5), usually 10 to 1,000 mL, preferably 50 to 400 mL.

The polymerization can also be carried out in the presence of additives. For example, an inorganic salt such as lithium chloride or calcium chloride may be added in order to obtain a compound having a large molecular weight. OF these, lithium chloride is preferable as the additive. The amount of the additive added is, based on the amount of the solvent, preferably 10% by mass or less, and more preferably 5% by mass or less.

The polymerization can be carried out by, for example, dissolving any one of the fluorinated compound (4) and the compound (5) in the solvent, adding the other compound to the resulting solution, and then carrying out the reaction while stirring under an inert atmosphere such as nitrogen. The polymerization temperature is preferably -50 to 150°C, more preferably 0 to 100°C, and further preferably 35 to 80°C. The polymerization time is preferably 0.1 to 50 hours, and more preferably 1 to 24 hours.

The average degree of polymerization of the repeating unit represented by Formula (1) can be adjusted by adjusting the molar ratio of the fluorinated compound (4) to the compound (5), the polymerization temperature, the polymerization time, the concentration of the polymerization solution, and the like.

Generally, a solution of the fluorinated amide compound is obtained by the production method described above. The obtained solution of the fluorinated amide compound may be used as it is for various purposes. Further, a highly pure fluorinated amide compound may be obtained by charging the obtained solution of the fluorinated amide compound into a poor solvent such as methanol or water to separate the fluorinated amide compound, and then purifying by a reprecipitation method to remove by-products, inorganic salts, and the like.

The fluorinated compound (4a-1) can be produced by reacting the compound (6a) represented by Formula (6a) with 1,8-diiodoperfluorooctane.

Formula (6a):

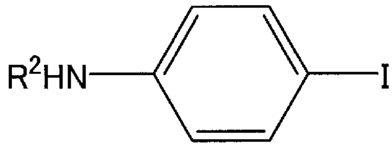

(In Formula (6a), $R^2$ is the same as in Formula (1).)

The reaction between the compound (6a) and 1,8-diiodoperfluorooctane can be carried out in a solvent. It is desirable that the solvent does not substantially react with the compound (6a) and 1,8-diiodoperfluorooctane, has the property of dissolving the compound (6a) and 1,8-diiodoperfluorooctane well, and is a good solvent for the obtained fluorinated compound (4a-1) . Such a solvent is not limited, and examples thereof include dimethyl sulfoxide (DMSO), N,N-dimethylformamide (DMF), N,N-dimethylacetamide (DMAc), N-methyl-2-pyrrolidone (NMP), 1,3-dimethylimidazolidone (DMI), sulfolane, tetrahydrofuran (THF), and acetone. Of these, dimethyl sulfoxide (DMSO) is preferable. The amount of these solvents used is, based on 0.1 mol of the compound (6a) or 1,8-diiodoperfluorooctane, usually 10 to 1,000 mL, preferably 50 to 400 mL.

The reaction of between the compound (6a) and 1,8-diiodoperfluorooctane can be carried out by, for example, dissolving any one of the compound (6a) and 1,8-diiodoperfluorooctane in the solvent, adding the other compound to the resulting solution, and then carrying out the reaction while stirring under an inert atmosphere such as nitrogen. The reaction temperature is preferably 50 to 150°C, and more preferably 100 to 140°C. The reaction time is preferably 1 to 50 hours, and more preferably 1 to 30 hours.

The reaction between the compound (6a) and 1,8-diiodoperfluorooctane may be carried out in the presence of a catalyst such as a copper and a copper compound.

After completion of the reaction, a highly pure fluorinated compound (4a-1) may be obtained by charging the reaction mixture into a poor solvent such as methanol or water to separate the fluorinated compound (4a-1), and then purifying by a reprecipitation method to remove by-products, inorganic salts, and the like.

The present disclosure also relates to a fluorinated compound (4B) represented by Formula (4B). Examples of the fluorinated compound (4B) include a fluorinated compound (4b) represented by Formula (4b). Both the fluorinated compound (4B) and the fluorinated compound (4b) are novel compounds.

Formula (4B):

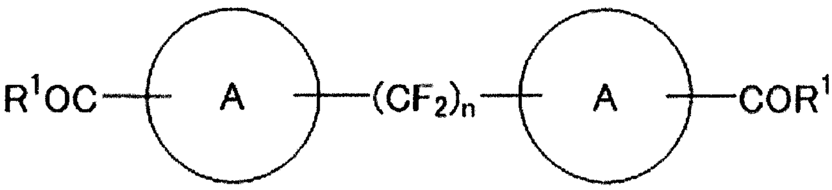

(In Formula (4B), n represents an integer of 4 to 8, ring A represents a hydrocarbon ring, and each $R^1$ independently represents OH, an optionally-substituted linear or branched chain alkoxy group, an optionally-substituted aromatic oxy group, or a halogen atom.)

In Formula (4B), n and $R^1$ are the same as n and $R^1$ in Formula (1) .

Ring A represents a hydrocarbon ring. The hydrocarbon ring may be a monocyclic or polycyclic ring, and may be an aliphatic hydrocarbon ring or an aromatic hydrocarbon ring. The aliphatic hydrocarbon ring may be a non-aromatic saturated or unsaturated hydrocarbon ring.

The position where the carbonyl group and the perfluoroalkylene group adjacent to the hydrocarbon ring of the ring A are bonded to the hydrocarbon ring is not limited. The carbonyl group and the perfluoroalkylene group may be bonded to any of the carbon atoms constituting the hydrocarbon ring.

The number of carbon atoms in the ring A is preferably 3 to 30, more preferably 5 or more, and further preferably 6 or more, and is more preferably 20 or less, and further preferably 14 or less.

The hydrocarbon ring of the ring A optionally has a substituent. Examples of the substituent include a halogen atom such as a fluorine atom, an alkyl group such as a methyl group, and an alkyl halide group such as a trifluoromethyl group.

Examples of the ring A include:

monocyclic saturated hydrocarbon rings, such as a cyclopropane ring, a cyclobutane ring, a cyclopentane ring, a

cyclohexane ring, a cycloheptane ring, a cyclooctane ring, a cyclononane ring, a cyclodecane ring, a cycloundecane ring, and a cyclododecane ring;

a monocyclic non-aromatic unsaturated hydrocarbon rings, such as a cyclopropene ring, a cyclobutene ring, a cyclopropene ring, a cyclohexene ring, a cycloheptene ring, and a cyclooctene ring;

polycyclic non-aromatic hydrocarbon rings, such as a norbornene ring, a norbornadiene ring, a decahydronaphthalene ring, a bicycloundecane ring, and a spirobicyclopentane ring;

aromatic hydrocarbon rings, such as a benzene ring, a naphthalene ring, a phenanthrene ring, an anthracene ring, a fluorene ring, a tetracene ring, a chrysene ring, a pyrene ring, a pentacene ring, a benzopyrene ring, a triphenylene ring, a perylene ring, a biphenyl ring, a terphenyl ring, a diphenylmethane ring, a diphenyl ether ring, a diphenyl sulfone ring, and a diphenyl ketone ring;

and the like.

Among these, the ring A is preferably a cyclohexane ring, a benzene ring, a naphthalene ring, an anthracene ring, or a biphenyl ring, and more preferably a benzene ring. These examples optionally have a substituent.

Next, a method for producing the fluorinated compound (4B) represented by Formula (4B) will be described. The fluorinated compound (4b) represented by Formula (4b) can also be produced by this production method. A fluorinated compound (4B) in which $R^1$ is OH can be produced by reacting a compound (10) represented by Formula (10) with a compound (7) represented by Formula (7) to obtain a compound (8) represented by Formula (8), and then oxidizing the obtained compound (8).

Formula (10):

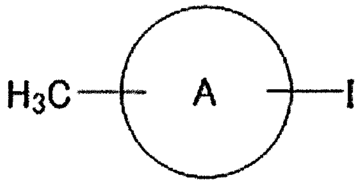

(In Formula (10), the ring A is the same as in Formula (4B).)

Formula (7) : $I\text{-}(CF_2)_n\text{-}I$

(In Formula (7), n is the same as in Formula (4B).)

Formula (8):

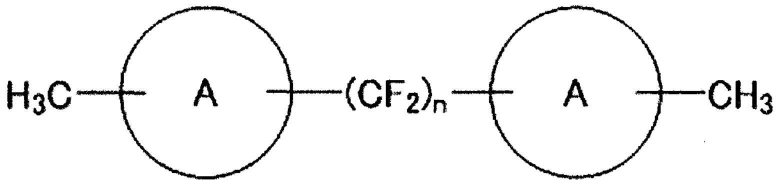

(In Formula (8), n and the ring A are the same as in Formula (4B).)

The compound (10) is preferably a compound represented by Formula (10-1), more preferably 4-iodotoluene or 3-iodotoluene.

Formula (10-1):

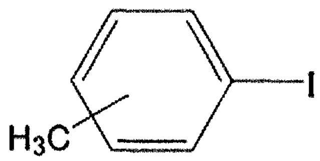

The compound (8) is preferably a compound represented by Formula (8-1).

Formula (8-1):

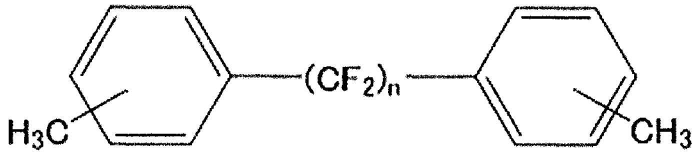

(In Formula (8-1), n is the same as in Formula (4B).)

Further preferably, the compound (8) is a compound represented by Formula (8-1a) or Formula (8-1b).

Formula (8-1a):

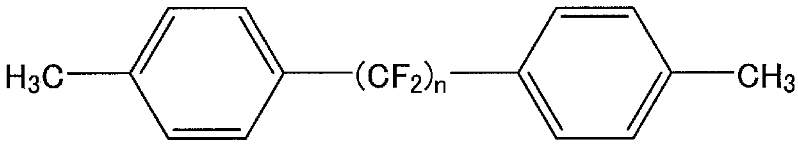

(In Formula (8-1a), n is the same as in Formula (4B).)

Formula (8-1b):

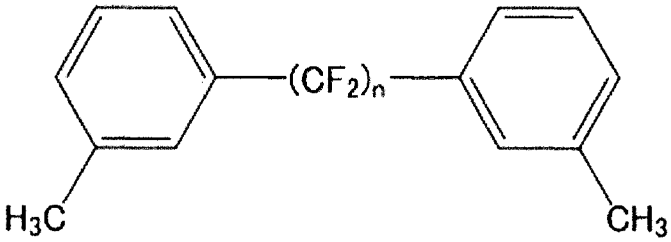

(In Formula (8-1b), n is the same as in Formula (4B).)

Further, by reacting the fluorinated compound (4B) in which $R^1$ is OH with a compound (9) represented by Formula (9), a fluorinated compound (4B) having a desired group represented by $R^1$ (for example, an alkoxy group or an aromatic oxy group) can be produced.

Formula (9): R-OH

(In Formula (9), R is an optionally-substituted linear or branched chain alkyl group or an optionally-substituted aromatic group.)

Further, by reacting the fluorinated compound (4B) in which $R^1$ is OH with a halogenating agent, a fluorinated compound (4B) in which $R^1$ is a halogen atom can be produced.

Further, by reacting the fluorinated compound (4B) in which $R^1$ is OH with a triazine chloride compound, a fluorinated compound (4B) in which $R^1$ is a triazinyloxy group can be produced.

The reaction between the compound (10) and the compound (7) can be carried out in a solvent. It is desirable that the solvent does not substantially react with the compound (10) and compound (7), has the property of dissolving the compound (10) and compound (7) well, and is a good solvent for the obtained compound (8). Such a solvent is not limited, and examples thereof include dimethyl sulfoxide (DMSO), N,N-dimethylformamide (DMF), N,N-dimethylacetamide (DMAc), N-methyl-2-pyrrolidone (NMP), 1,3-dimethylimidazolidone (DMI), sulfolane, tetrahydrofuran (THF), and acetone. Of these, dimethyl sulfoxide (DMSO) is preferable. The amount of these solvents used is, based on 0.1 mol of the compound (10) or compound (7), usually 10 to 1,000 mL, preferably 50 to 400 mL.

The reaction between the compound (10) and the compound (7) can be carried out by, for example, dissolving any one of the compound (10) and compound (7) in the solvent, adding the other compound to the resulting solution, and then carrying out the reaction while stirring under an inert atmosphere such as nitrogen. The reaction temperature is preferably 50 to 150°C, and more preferably 100 to 140°C. The reaction time is preferably 1 to 50 hours, and more preferably 1 to 30 hours.

The reaction between the compound (10) and the compound (7) may be carried out in the presence of a catalyst

such as copper or a copper compound.

The oxidation of the compound (8) can be performed using, for example, an oxidizing agent such as $CrO_3$ or $KMnO_4$. Further, the oxidation of the compound (8) is preferably carried out in the presence of an acidic compound such as sulfuric acid or acetic acid.

Examples of the halogenating agent used in the reaction between the fluorinated compound (4B) in which $R^1$ is OH and the halogenating agent include thionyl chloride, phosphorus trichloride, phosphorus pentachloride, and the like. The reaction temperature may be, for example, 20 to 100°C. The reaction between the fluorinated compound (4B) and the halogenating agent can also be carried out in a solvent. Examples of the solvent include an ether solvent such as diethyl ether and tetrahydrofuran.

The reaction between the fluorinated compound (4B) in which $R^1$ is OH and the triazine chloride compound can be carried out in a solvent in the presence of N-methylmorpholine (NMM). As a result of this reaction, a triazine-based active diester can be synthesized. It is desirable that the solvent does not substantially react with the fluorinated compound (4B), the triazine chloride compound, and N-methylmorpholine (NMM), has the property of dissolving the fluorinated compound (4B), the triazine chloride compound, and N-methylmorpholine (NMM) well, and is a good solvent for the obtained triazine-based active diester. Such a solvent is not limited, and examples thereof include dimethyl sulfoxide (DMSO), N,N-dimethylformamide (DMF), N,N-dimethylacetamide (DMAc), N-methyl-2-pyrrolidone (NMP), 1,3-dimethylimidazolidone (DMI), sulfolane, tetrahydrofuran (THF), and acetone. Of these, N-methyl-2-pyrrolidone (NMP) is preferable. The amount of these solvents used is, based on 0.1 mol of the fluorinated compound (4B) or triazine chloride compound, usually 10 to 1,000 mL, preferably 50 to 400 mL.

The reaction between the fluorinated compound (4B) in which $R^1$ is OH and the triazine chloride compound can be carried out by, for example, dissolving any one of the fluorinated compound (4B) in which $R^1$ is OH and the triazine chloride compound in the solvent, adding the other compound to the resulting solution, and then carrying out the reaction while stirring under an inert atmosphere such as nitrogen. The reaction temperature is preferably 0 to 150°C, more preferably 0 to 100°C, and further preferably 0 to 50°C. The reaction time is preferably 1 to 50 hours, and more preferably 1 to 30 hours.

<Method for producing fluorinated nitrogen-containing heterocyclic ring-containing compound>

The fluorinated nitrogen-containing heterocyclic ring-containing compound of the present disclosure can be suitably produced by obtaining a fluorinated amide compound by the production method described above, and then cyclodehydrating the fluorinated amide compound. Further, when producing the fluorinated amide compound, in a case in which the polymerization of the fluorinated compound (4) and the compound (5) is carried out in a heated state, a part or all of the compound undergoes cyclodehydration, which may result the fluorinated nitrogen-containing heterocyclic ring-containing compound of the present disclosure being obtained as a part or all of the product. That is, the present disclosure also includes mixtures of the fluorinated amide compound and the fluorinated nitrogen-containing heterocyclic ring-containing compound.

The cyclodehydration of the fluorinated amide compound can be carried out by heating the fluorinated amide compound. The heating temperature for the cyclodehydration is preferably 110 to 450°C, and more preferably 150 to 350°C. The heating time is preferably 0.1 to 10 hours, and more preferably 0.5 to 8 hours. The cyclodehydration can be carried out in air, in a nitrogen or argon atmosphere, or under reduced pressure.

The fluorinated amide compound and the fluorinated nitrogen-containing heterocyclic ring-containing compound of the present disclosure have a low dielectric constant and a low dielectric loss, and thus can be suitably used as a low dielectric material.

The fluorinated amide compound and the fluorinated nitrogen-containing heterocyclic ring-containing compound of the present disclosure have a low dielectric constant and a low dielectric loss, and thus can be suitably used as a semiconductor package substrate, a flexible printed circuit board, and a rigid printed circuit board.

The fluorinated amide compound and the fluorinated nitrogen-containing heterocyclic ring-containing compound of the present disclosure have a low dielectric constant and a low dielectric loss, and thus they can be suitably used for a semiconductor package wiring board, a flexible printed wiring board, a rigid printed wiring board, a TAB tape, a COF tape, metal wiring, and the like, as well as a material of electronic components and electronic devices, such as a cover substrate of, for example, a chip member such as metal wiring or an IC chip, a liquid crystal display, an organic electroluminescence display, electronic paper, an interlayer insulating film of a solar cell or the like, a base substrate, an adhesive sheet, a prepreg, and a primer.

The fluorinated amide compound and the fluorinated nitrogen-containing heterocyclic ring-containing compound of the present disclosure have a low dielectric constant in particular at high frequencies and a low dielectric loss, and thus can be suitably used as a material for electronic components and electronic devices that utilize high frequencies, in particular microwaves of 3 to 30 GHz. For example, the fluorinated amide compound and the fluorinated nitrogen-containing heterocyclic ring-containing compound of the present disclosure can be suitably used as a material for an

insulating plate of a high-frequency circuit, an insulating material for connecting components, a printed circuit board, a base of a high-frequency vacuum tube, an antenna cover, a coated electric wire of, for example, a coaxial cable or a LAN cable, and the like. Further, the fluorinated amide compound and the fluorinated nitrogen-containing heterocyclic ring-containing compound of the present disclosure can be suitably used as a material for devices such as satellite communication equipment and mobile phone base stations that use microwaves of 3 to 30 GHz.

The printed circuit board is not limited, and examples thereof include printed wiring boards for electronic circuits of mobile phones, various types of computers, communication devices, and the like.

The coaxial cable is not limited, and examples thereof include coaxial cables having a structure in which an inner conductor, an insulating coating layer, an outer conductor layer, and a protective coating layer are laminated in that order from the core portion to the outer peripheral portion.

The fluorinated amide compound and the fluorinated nitrogen-containing heterocyclic ring-containing compound of the present disclosure have a low dielectric constant and a low dielectric loss, also have excellent heat resistance, solvent solubility, electrical insulation, colorless transparency, and flexibility, and can be easily reduced in thickness, and thus can be suitably used for an interlayer insulating film, a film, an adhesive sheet, a prepreg, a primer, a polyelectrolyte film, a resist material, and the like. Of those, use as a film is suitable.

The film can be produced by molding the fluorinated amide compound or fluorinated nitrogen-containing heterocyclic ring-containing compound of the present invention by a known film molding method such as extrusion molding, calendar molding, or solution casting. Further, by casting and heating the solution containing the fluorinated amide compound of the present invention, it is also possible to form a film simultaneously with the formation of the fluorinated nitrogen-containing heterocyclic ring-containing compound by cyclodehydration of the fluorinated amide compound. Moreover, the film may be subjected to a sandblasting treatment, a corona treatment, a plasma treatment, an etching treatment, or the like.

Embodiments of the present disclosure have been described above, but it will be understood that various modifications can be made to the embodiments and details thereof without departing from the gist and scope of the claims.

EXAMPLES

Next, the embodiments of the present disclosure will be described with reference to examples, but the present disclosure is not limited to such examples.

The numerical values in the examples were measured by the following methods.

(1) GPC: High-speed GPC System HLC-8220GPC, manufactured by Tosoh Corporation (column: Tosoh TSKgel ($\alpha$-M), column temperature: 45°C, detector: UV-8020, wavelength 254 nm, eluent: N-methyl-2-pyrrolidone (NMP) (including 0.01 mol/L lithium bromide), calibration curve:
standard polystyrene, column flow velocity: 0.2 mL/min)
(2) Infrared spectrum (FT-IR): FT/IR-4200, manufactured by JASCO Corporation
(3) Nuclear magnetic resonance spectrum (NMR): AC400P, manufactured by BRUKER
(4) Thermogravimetric analysis (TGA): TG/DTA7300, manufactured by Hitachi High-Tech Science Corporation, temperature-increasing rate 10 °C/min
(5) Differential scanning calorimetry (DSC): DSC7000, manufactured by Hitachi High-Tech Science Corporation, temperature-increasing rate 10 °C/min
(6) Thermomechanical analysis (TMA) : TMA7000, manufactured by Hitachi High-Tech Science Corporation, temperature-increasing rate 10 °C/min
(7) Dynamic viscoelasticity measurement (DMA) : DMA7100, manufactured by Hitachi High-Tech Science Corporation, temperature-increasing rate 2 °C/min
(8) Tensile test: Autograph AGS-D type, manufactured by Shimadzu Corporation, tensile speed 10 mm/min
(9) Ultraviolet-visible spectrophotometer: UV-1800, manufactured by Shimadzu Corporation
(10) Refractive index measurement: Metricon Model 2010/M PRISM COUPLER
(11) Dielectric constant measurement: Dielectric constant / dielectric tangent measuring device (cavity resonator type, 10 GHz) manufactured by AET

<Synthesis example 1>

1,6-Bis(4-aminophenyl)dodecafluorohexane (APDF6)

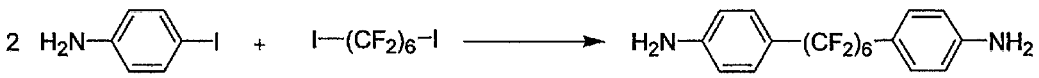

APDF6

4-iodoaniline (15 g, 69 mmol) and DMSO (60 mL) were charged into a 100 mL eggplant flask equipped with a stirrer, a Dimroth condenser, and a three-way cock, and made to dissolve. Then, the mixture was quickly charged with copper powder (21.6 g, 342 mmol) so as not to oxidize in air, and quickly charged with 1,6-diiodoperfluorohexane (18.9 g, 33 mmol) before it deliquesced. Then, the temperature was raised to 120°C, and the mixture was reacted while stirring for 24 hours at 120°C. After completion of the reaction, the mixture was cooled to room temperature and suction filtered to recover the DMSO solution. The DMSO was distilled off from the DMSO solution by vacuum distillation to obtain a crude product. The crude product was dissolved in dichloromethane, washed with distilled water, the dichloromethane solution was recovered, and the resulting product was dried over anhydrous sodium sulfate overnight. The dichloromethane was distilled off with an evaporator to obtain a yellow crude product. The yellow crude product was recrystallized from hexane, and dried under reduced pressure at room temperature. The product was dissolved in methanol and decolorized with activated carbon. After removing the methanol, the product was dried under reduced pressure at 60°C overnight to obtain APDF6 (yield amount: 10.8 g, yield: 65%) in the form of a yellow powder.

The structure was confirmed by FT-IR, $^{1}$H-NMR, $^{13}$C-NMR, $^{19}$F-NMR, and elemental analysis.

FT-IR (KBr, $cm^{-1}$): 3444 (N-H), 3057 (C-H), 1609 (C=C), 1185 (C-F)
$^{1}$H-NMR (DMSO-$d_6$, ppm): 5.82 (s, 4H, $NH_2$), 6.62 (d, 4H, Ar-H), 7.20 (d, 4H, Ar-H)
$^{13}$C-NMR (DMSO-$d_6$, ppm): 113.0, 127.6, 152.4
$^{19}$F-NMR (DMSO-$d_6$, ppm): -122.2, -121.5, -108.0
Melting point: 79.4°C (DSC)
Elemental analysis: Calculated C, 44.64%; H, 2.50%; N, 5.78%
Measured C, 44.66%; H, 2.72%; N, 5.49%

<Example 1>

1,8-Bis(4-aminophenyl)hexadecafluorooctane (APHF8)

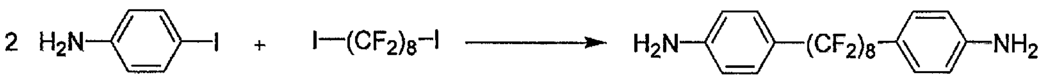

APHF8

4-iodoaniline (7.5 g, 34 mmol) and DMSO (20 mL) were charged into a 100 mL eggplant flask equipped with a stirrer, a Dimroth condenser, and a three-way cock, and made to dissolve. Then, the mixture was quickly charged with copper powder (10 g, 172 mmol) so as not to oxidize in air, and quickly charged with 1,8-diiodoperfluorooctane (11.6 g, 17 mmol) before it deliquesced. Then, the temperature was raised to 120°C, and the mixture was reacted while stirring for 24 hours at 120°C. After completion of the reaction, the mixture was cooled to room temperature and suction filtered to recover the DMSO solution. The DMSO was distilled off from the DMSO solution by vacuum distillation to obtain a crude product. The crude product was dissolved in dichloromethane, washed with distilled water, the dichloromethane solution was recovered, and the resulting product was dried over anhydrous sodium sulfate overnight. The dichloromethane was distilled off with an evaporator, and 8.4 g (80%) of a crude product was collected. The crude product was recrystallized from hexane, and dried under reduced pressure at room temperature. The product was dissolved in methanol and decolorized with activated carbon. The methanol was removed to obtain APHF8 (yield amount: 5.9 g, yield: 58%) in the form of a yellow powder.

The structure was confirmed by FT-IR, $^{1}$H-NMR, $^{13}$C-NMR, and $^{19}$F-NMR.

FT-IR (KBr, $cm^{-1}$): 3489-3392 (N-H), 3050 (C-H), 1581 (C=C), 1259-1145 (C-F) $^{1}$H-NMR (DMSO-$d_6$, ppm): 5.86 (s, 4H, $NH_2$), 6.66 (d, 4H, Ar-H), 7.22 (d, 4H, Ar-H)
$^{13}$C-NMR (DMSO-$d_6$, ppm): 113.0, 127.8, 152.4
$^{19}$F-NMR (DMSO-$d_6$, ppm): -121.7, -121.1, -107.5
Melting point: 85.8°C (DSC)
Elemental analysis: Calculated C, 41.16%; H, 2.08%; N, 4.80%

Measured C, 41.23%; H, 2.28%; N, 4.85%

<Synthesis example 2>

1,6-Bis(p-tolyl)dodecafluorohexane (TDF6)

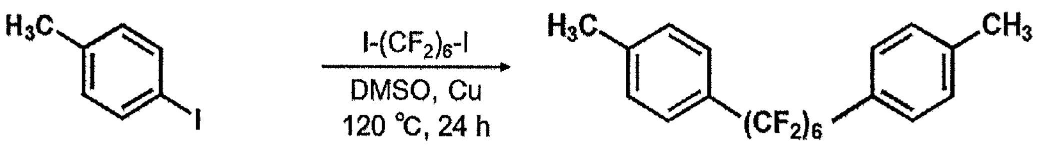


TDF6

4-Iodotoluene (8.72 g, 40 mmol) and DMSO (20 mL) were charged into a 100 mL three-necked flask equipped with a stirrer, a condenser, and a nitrogen introduction tube, and made to dissolve. Next, 1,6-diiodoperfluorohexane (11.08 g, 20 mmol) and copper powder (12.69 g, 200 mmol) were added, the temperature was increased in steps to 120°C, and the mixture was reacted for 24 hours at 120°C. After completion of the reaction, the mixture was allowed to cool to room temperature, the copper powder was removed by suction filtration, and the DMSO was distilled off by vacuum distillation. The obtained crude product was dissolved in diethyl ether (100 mL), and washed with distilled water. The organic layer was separated and dehydrated overnight with anhydrous sodium sulfate. The diethyl ether was distilled off with an evaporator to obtain a white product (TDF6) (yield amount: 8.59 g, yield: 89%).

The physical properties of the obtained product are shown below.

$^{1}$H-NMR ($CDCl_3$, ppm): 2.34 (s, 6H, $CH_3$), 7.41 (d, 4H, Ar-H), 7.51 (d, 4H, Ar-H)
$^{13}$C-NMR ($CDCl_3$, ppm): 20.86, 126.46, 129.67, 136.58, 136.67, 138.60, 138.71, 142.90
$^{19}$F-NMR ($CDCl_3$, ppm): -109.72, -121.57, -122.13

<Example 2>

1,6-Bis(4-carboxyphenyl)dodecafluorohexane (CPDF6)

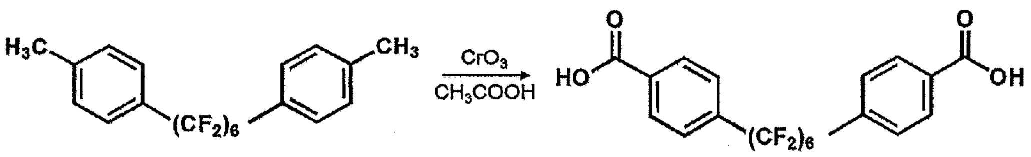


CPDF6

TDF6 (14.47 g, 30 mmol), acetic acid (150 mL), and concentrated sulfuric acid (20 mL) were charged into a 500 mL three-necked flask equipped with a stirrer, a nitrogen introduction tube, a thermometer, and a dropping funnel, made to dissolve, and the resulting mixture was cooled to 0°C. A solution prepared by dissolving chromium oxide (10.00 g, 100 mmol) in acetic anhydride (50 mL) was added dropwise, and after the addition was completed, the mixture was reacted for 12 hours at room temperature while stirring. After completion of the reaction, the reaction solution was charged into distilled water (1 L) to precipitate a product. The product was collected by suction filtration, and dried under reduced pressure for 12 hours at 100°C. The yield amount obtained was 15.9 g (98% yield). The dried product was recrystallized from a mixed solvent of DMF / distilled water, and dried under reduced pressure for 12 hours at 100°C to obtain a product in the form of a white powder (yield amount: 6.35 g, yield: 39%).

The physical properties of the obtained product are shown below.

$^{1}$H-NMR (DMSO-$d_6$, ppm): 7.80 (d, 4H, Ar-H), 8.13 (d, 4H, Ar-H), 13.50 (s, 1H, COOH)
$^{13}$C-NMR (DMSO-$d_6$, ppm): 127.15, 129.93, 131.19, 134.74, 136.57, 136.66, 138.70, 166.23
$^{19}$F-NMR (DMSO-$d_6$, ppm): -110.41, -121.57, -122.04

<Example 3>

Bis(4,6-dimethoxy-1,3,5-triazin-2-yl)-p-dodecafluorohexylenedibenzoate (p-DFBBT)

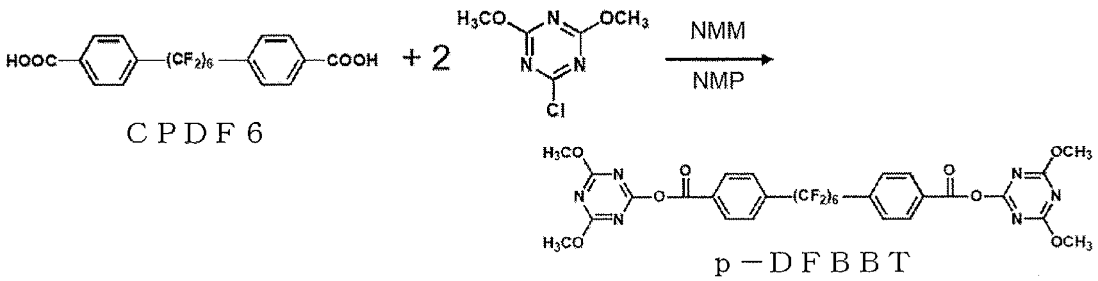


CPDF6

p-DFBBT

CPDF6 (27.11 g, 50 mmol), chlorodimethoxytriazine (CDMT) (17.51 g, 100 mmol), and NMP (400 mL) were charged into a 500 mL three-necked flask equipped with a stirrer, a nitrogen introduction tube, and a thermometer, made to dissolve, and the resulting mixture was cooled to 0°C. Then, N-methylmorpholine (NMM) (11.59 mL, 110 mmol) was added, and the mixture was reacted for 1 hour at 0°C. After completion of the reaction, the reaction solution was charged into 400 mL of an aqueous solution adjusted to pH 3 using acetic acid to precipitate a product. The product was collected by suction filtration and dried under reduced pressure for 12 hours at 50°C. The crude yield amount was 32.8 g (crude yield 80%). The dried crude product was recrystallized from a mixed solvent of chloroform / hexane, and dried under reduced pressure for 12 hours at 50°C to obtain a product in the form of a white powder (yield amount: 21.33 g, yield: 52%) .

The physical properties of the obtained product are shown below.

$^1$H-NMR ($CDCl_3$, ppm): 4.09 (s, 12H, $CH_3$), 7.76 (d, 4H, Ar-H), 8.30 (s, 4H, Ar-H)
$^{13}$C-NMR ($CDCl_3$, ppm): 56.20, 127.64, 130.90, 131.61, 137.01, 137.10, 139.19, 161.54, 170.70, 171.13, 174.39
$^{19}$F-NMR ($CDCl_3$, ppm): -112.65, -122.53, -123.10

<Example 4>

Fluorinated amide compound and fluorinated nitrogen-containing heterocyclic ring-containing compound (imide compound: APDF6-BPDA)

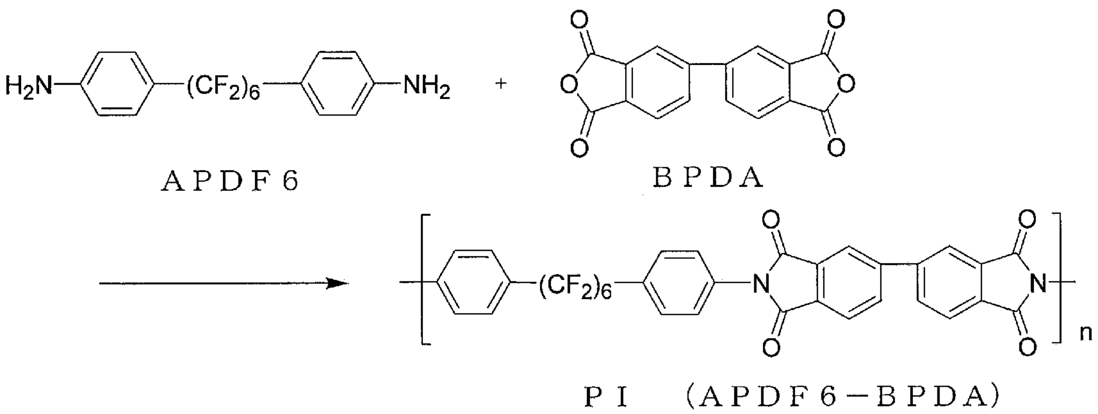

APDF6

BPDA

PI (APDF6-BPDA)

A 100 mL three-necked flask equipped with a stirring rod, a stirring blade, a Dimroth condenser, and a nitrogen introduction tube was heated and dried with a burner, then APDF6 (0.726 g, 1.50 mmol) and distilled NMP (3 mL) were added, and the mixture was stirred to dissolve. Then, 3,3',4,4'-biphenyltetracarboxylic acid dianhydride (BPDA) (0.441 g, 1.50 mmol) was added, the temperature was gradually raised, and the mixture was reacted by stirring for 18 hours at 60°C to obtain a solution of the fluorinated amide compound. The logarithmic viscosity ($\eta_{inh}$) of the fluorinated amide compound was 0.72 dL/g (NMP solution having a concentration of 0.5 g/dL, measured at 30°C). The solution was cooled

to room temperature, distilled NMP (2 mL) was added, the mixture was stirred to dilute the concentration of the entire solution, and the solution was cast on a glass plate. The cast mixture was dried under reduced pressure for 3 hours at room temperature, then dried under reduced pressure in a program consisting of 6 hours at 60°C, 1 hour at 100°C, 1 hour at 150°C, 1 hour at 200°C, and 1 hour at 250°C to obtain a yellow opaque fluorinated nitrogen-containing heterocyclic ring-containing compound (imide compound) film (thickness 25 μm).

The physical properties of the obtained fluorinated nitrogen-containing heterocyclic ring-containing compound (imide compound) film are shown below.

FT-IR (KBr, $cm^{-1}$): 1,778 (C=O), 1,734 (C=O), 1,389 (C-N), 1,185 (C-F), 740 (imide ring)
Solubility: Insoluble in organic solvents
5% Weight loss temperature: 520°C (in air), 555°C (in nitrogen) (TGA)
10% Weight loss temperature: 550°C (in air), 576°C (in nitrogen) (TGA) Carbonization yield: 53% (800°C in nitrogen) (TGA)
Glass transition temperature: 210°C (DMA)
Thermal expansion coefficient: 43 ppm/°C
Cut-off wavelength: 389 nm
Transmission at 500 nm: 28%
Average refractive index ($n_{ave}$): 1.579 (d line)
Dielectric constant (ε) calculated from refractive index: 2.72 ($\varepsilon = 1.10 \times n_{ave}^2$)

<Example 5>

Fluorinated amide compound and fluorinated nitrogen-containing heterocyclic ring-containing compound (imide compound: APDF6-6FDA)

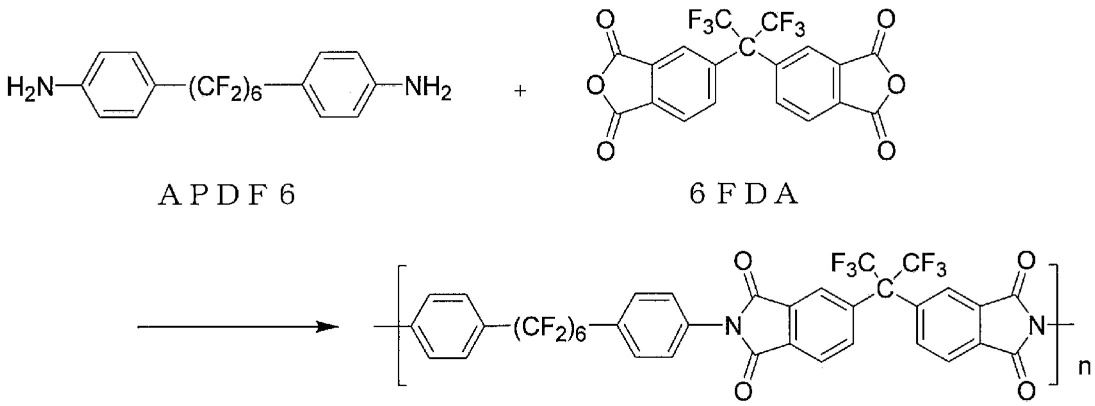

APDF6

6FDA

PI (APDF6-6FDA)

A 100 mL three-necked flask equipped with a stirring rod, a stirring blade, a Dimroth condenser, and a nitrogen introduction tube was heated and dried with a burner, then APDF6 (0.726 g, 1.50 mmol) and distilled NMP (3 mL) were added, and the mixture was stirred to dissolve. Then, 4,4'-(hexafluoroisopropylidene)diphthalic acid dianhydride (6FDA) (0.666 g, 1.50 mmol) was added, the temperature was gradually raised, and the mixture was reacted by stirring for 18 hours at 60°C to obtain a solution of the fluorinated amide compound. The logarithmic viscosity ($\eta_{inh}$) of the fluorinated amide compound was 0.32 dL/g (NMP solution having a concentration of 0.5 g/dL, measured at 30°C). The fluorinated amide compound was soluble in NMP, DMAc, γ-butyrolactone, acetone, methanol, ethanol, ethyl acetate, THF, and cyclohexanone. The solution was cooled to room temperature, distilled NMP (2 mL) was added, the mixture was stirred to dilute the concentration of the entire solution, and the solution was cast on a glass plate. The cast mixture was dried under reduced pressure for 3 hours at room temperature, then dried under reduced pressure in a program consisting of 6 hours at 60°C, 1 hour at 100°C, 1 hour at 150°C, 1 hour at 200°C, and 1 hour at 250°C to obtain a pale yellow transparent fluorinated nitrogen-containing heterocyclic ring-containing compound (imide compound) film (thickness 22 μm).

The physical properties of the obtained fluorinated nitrogen-containing heterocyclic ring-containing compound

(imide compound) film are shown below.

FT-IR (KBr, $cm^{-1}$): 1,789 (C=O), 1,725 (C=O), 1,376 (C-N), 1,199 (C-F), 745 (imide ring)
$^1$H-NMR ($CDCl_3$, ppm): 7.65 (d, 4H, Ar-H), 7.76 (d, 4H, Ar-H), 7.91 (d, 2H, Ar-H), 7.96 (s, 2H, Ar-H), 8.08 (d, 2H, Ar-H)
$^{19}$F-NMR ($CDCl_3$, ppm): -126.8, -126.4, -115.9, -68.4
Logarithmic viscosity ($\eta_{inh}$): 0.52 dL/g (NMP solution having a 0.5 g/dL concentration, measured at 30°C)
GPC: Number average molecular weight (Mn) 47,000, molecular weight distribution (Mw/Mn) 2.5
Solubility: Dissolved in NMP, DMAc, DMF, DMI, THF, chloroform, acetone, methanol, ethyl acetate, THF, and cyclohexane at room temperature 5% Weight loss temperature: 509°C (in air), 524°C (in nitrogen) (TGA) 10% Weight loss temperature: 527°C (in air), 544°C (in nitrogen) (TGA) Carbonization yield: 52% (800°C in nitrogen) (TGA)
Glass transition temperature: 221°C (DSC), 215°C (DMA), 213°C (TMA) Thermal expansion coefficient (CTE): 92 ppm/°C
Tensile breaking strength: 58 MPa
Elongation at break: 9.3%
Tensile elasticity: 1.6 GPa
Cut-off wavelength: 337 nm
Transmission at 500 nm: 80%
Average refractive index ($n_{ave}$): 1.526 (d line)
Dielectric constant ($\varepsilon$) calculated from refractive index: 2.55 ($\varepsilon = 1.10 \times n_{ave}^2$)
Dielectric constant (Dk) at 10 GHz: 2.54
Dielectric tangent (Df) at 10 GHz: 0.0022

<Example 6>

Fluorinated amide compound and fluorinated nitrogen-containing heterocyclic ring-containing compound (imide compound: APDF6-BPDA/6FDA (50/50))

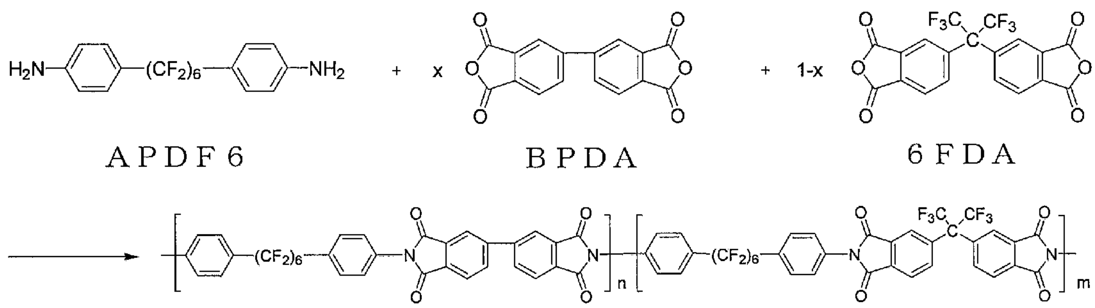

APDF6 BPDA 6FDA

PI (APDF6-BPDA/6FDA)

A 100 mL three-necked flask equipped with a stirring rod, a stirring blade, a Dimroth condenser, and a nitrogen introduction tube was heated and dried with a burner, then APDF6 (0.726 g, 1.50 mmol) and distilled NMP (3 mL) were added, and the mixture was stirred to dissolve. Then, 3,3',4,4'-biphenyltetracarboxylic acid dianhydride (BPDA) (0.221 g, 0.75 mmol) and 4,4'-(hexafluoroisopropylidene)diphthalic acid dianhydride (6FDA) (0.333 g, 0.75 mmol) were added, the temperature was gradually raised, and the mixture was reacted by stirring for 18 hours at 60°C to obtain a solution of the fluorinated amide compound. The logarithmic viscosity ($\eta_{inh}$) of the fluorinated amide compound was 0.35 dL/g (NMP solution having a concentration of 0.5 g/dL, measured at 30°C). The solution was cooled to room temperature, distilled NMP (2 mL) was added, the mixture was stirred to dilute the concentration of the entire solution, and the solution was cast on a glass plate. The cast mixture was dried under reduced pressure for 3 hours at room temperature, then dried under reduced pressure in a program consisting of 6 hours at 60°C, 1 hour at 100°C, 1 hour at 150°C, 1 hour at 200°C, and 1 hour at 250°C in a reduced-pressure dryer to obtain a yellow opaque fluorinated nitrogen-containing heterocyclic ring-containing compound (imide compound) film (thickness 35 μm).

The physical properties of the obtained fluorinated nitrogen-containing heterocyclic ring-containing compound (imide compound) film are shown below.

FT-IR (KBr, $cm^{-1}$): 1,792 (C=O), 1,741 (C=O), 1,380 (C-N), 1,179 (C-F), 721 (imide ring)

Logarithmic viscosity ($\eta_{inh}$): 0.43 dL/g (NMP solution having a 0.5 g/dL concentration, measured at 30°C)
Solubility: Dissolved in NMP, DMAc, and DMF at room temperature
5% Weight loss temperature: 503°C (in air), 526°C (in nitrogen) (TGA) 10% Weight loss temperature: 526°C (in air), 550°C (in nitrogen) (TGA)
Carbonization yield: 49% (800°C in nitrogen) (TGA)
Glass transition temperature: 205°C (DSC), 208°C (DMA), 210°C (TMA) Thermal expansion coefficient (CTE): 73 (ppm/°C)
Cut-off wavelength: 365 nm
Transmission at 500 nm: 73%
Average refractive index ($n_{ave}$): 1.562 (d line)
Dielectric constant ($\varepsilon$) calculated from refractive index: 2.68 ($\varepsilon = 1.10 \times n_{ave}^2$)
Dielectric constant (Dk) at 10 GHz: 2.58
Dielectric tangent (Df) at 10 GHz: 0.0025

<Example 7>

Fluorinated amide compound and fluorinated nitrogen-containing heterocyclic ring-containing compound (imide compound: APHF8-BPDA)

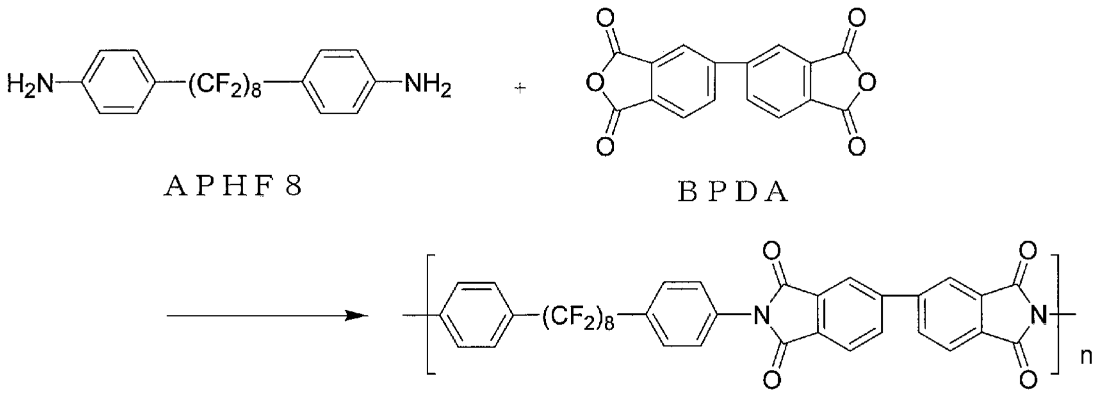
APHF8
BPDA

PI (APHF8-BPDA)

A 100 mL three-necked flask equipped with a stirring rod, a stirring blade, a Dimroth condenser, and a nitrogen introduction tube was heated and dried with a burner, then APHF8 (0.886 g, 1.50 mmol) and distilled NMP (3 mL) were added, and the mixture was stirred to dissolve. Then, 3,3',4,4'-biphenyltetracarboxylic acid dianhydride (BPDA) (0.441 g, 1.50 mmol) was added, the temperature was gradually raised, and the mixture was reacted by stirring for 18 hours at 60°C to obtain a solution of the fluorinated amide compound. The logarithmic viscosity ($\eta_{inh}$) of the fluorinated amide compound was 0.54 dL/g (NMP solution having a concentration of 0.5 g/dL, measured at 30°C). The solution was cooled to room temperature, distilled NMP (2 mL) was added, the mixture was stirred to dilute the concentration of the entire solution, and the solution was cast on a glass plate. The cast mixture was dried under reduced pressure for 3 hours at room temperature, then dried under reduced pressure in a program consisting of 6 hours at 60°C, 1 hour at 100°C, 1 hour at 150°C, 1 hour at 200°C, and 1 hour at 250°C to obtain a yellow opaque fluorinated nitrogen-containing heterocyclic ring-containing compound (imide compound) film (thickness 63 $\mu$m).
The physical properties of the obtained fluorinated nitrogen-containing heterocyclic ring-containing compound (imide compound) film are shown below.

FT-IR (KBr, $cm^{-1}$): 1,782 (C=O), 1,725 (C=O), 1,375 (C-N), 1,205 (C-F), 739 (imide ring)
Solubility: Insoluble in organic solvents
5% Weight loss temperature: 521°C (in air), 545°C (in nitrogen) (TGA) 10% Weight loss temperature: 547°C (in air), 564°C (in nitrogen) (TGA) Carbonization yield: 48% (800°C in nitrogen) (TGA)
Glass transition temperature: 190°C (DMA)
Average refractive index ($n_{ave}$): 1.551 (d line)
Dielectric constant ($\varepsilon$) calculated from refractive index: 2.65 ($\varepsilon = 1.10 \times n_{ave}^2$)

Dielectric constant (Dk) at 10 GHz: 2.72
Dielectric tangent (Df) at 10 GHz: 0.0014

<Example 8>

Fluorinated amide compound and fluorinated nitrogen-containing heterocyclic ring-containing compound (imide compound: APHF8-6FDA)

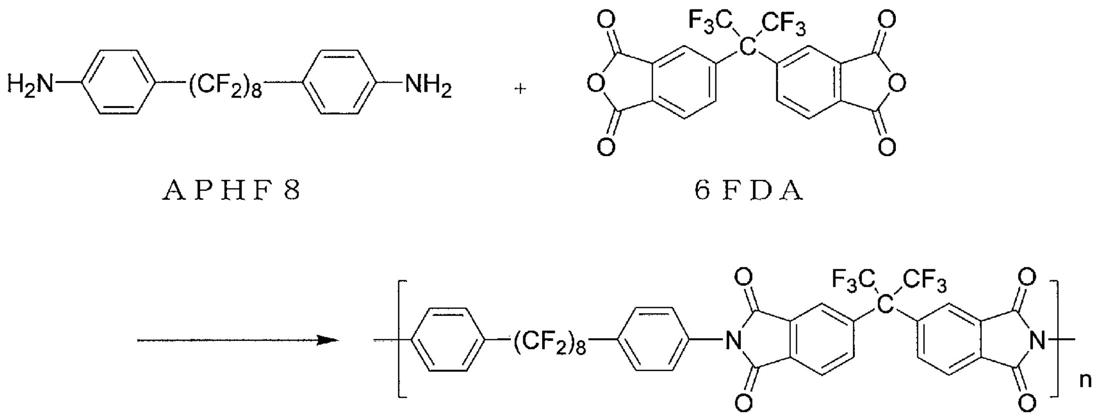

APHF8 6FDA

PI (APHF8-6FDA)

A 100 mL three-necked flask equipped with a stirring rod, a stirring blade, a Dimroth condenser, and a nitrogen introduction tube was heated and dried with a burner, then APHF8 (0.886 g, 1.50 mmol) and distilled NMP (3 mL) were added, and the mixture was stirred to dissolve. Then, 4,4'-(hexafluoroisopropylidene)diphthalic acid dianhydride (6FDA) (0.666 g, 1.50 mmol) was added, the temperature was gradually raised, and the mixture was reacted by stirring for 18 hours at 60°C to obtain a solution of the fluorinated amide compound. The logarithmic viscosity ($\eta_{inh}$) of the fluorinated amide compound was 0.43 dL/g (NMP solution having a concentration of 0.5 g/dL, measured at 30°C). The fluorinated amide compound was soluble in NMP, DMAc, γ-butyrolactone, acetone, methanol, ethanol, ethyl acetate, THF, and cyclohexanone. The solution was cooled to room temperature, distilled NMP (2 mL) was added, the mixture was stirred to dilute the concentration of the entire solution, and the solution was cast on a glass plate. The cast mixture was dried under reduced pressure for 3 hours at room temperature, then dried under reduced pressure in a program consisting of 6 hours at 60°C, 1 hour at 100°C, 1 hour at 150°C, 1 hour at 200°C, and 1 hour at 250°C to obtain a pale yellow transparent fluorinated nitrogen-containing heterocyclic ring-containing compound (imide compound) film (thickness 35 μm).

The physical properties of the obtained fluorinated nitrogen-containing heterocyclic ring-containing compound (imide compound) film are shown below.

FT-IR (KBr, $cm^{-1}$): 1,793 (C=O), 1,742 (C=O), 1,381 (C-N), 1,180 (C-F), 720 (imide ring)
Logarithmic viscosity ($\eta_{inh}$): 0.51 dL/g (NMP solution having a 0.5 g/dL concentration, measured at 30°C)
GPC: Number average molecular weight (Mn) 27,000, molecular weight distribution (Mw/Mn) 1.6
Solubility: Dissolved in NMP, DMAc, DMF, DMI, THF, and chloroform at room temperature
5% Weight loss temperature: 497°C (in air), 524°C (in nitrogen) (TGA) 10% Weight loss temperature: 516°C (in air), 544°C (in nitrogen) (TGA) Carbonization yield: 49% (800°C in nitrogen) (TGA)
Glass transition temperature: 194°C (DSC), 190°C (DMA), 188°C (TMA) Thermal expansion coefficient (CTE): 85 (ppm/°C)
Tensile breaking strength: 59 MPa
Elongation at break: 4.8%
Tensile elasticity: 1.7 GPa
Cut-off wavelength: 330 nm
Transmission at 500 nm: 83%
Average refractive index ($n_{ave}$): 1.511 (d line)
Dielectric constant (ε) calculated from refractive index: 2.51 ($\varepsilon = 1.10 \times n_{ave}^2$)
Dielectric constant (Dk) at 10 GHz: 2.39

Dielectric tangent (Df) at 10 GHz: 0.0016

<Example 9>

Fluorinated amide compound (6FTA-p-DFBBT)

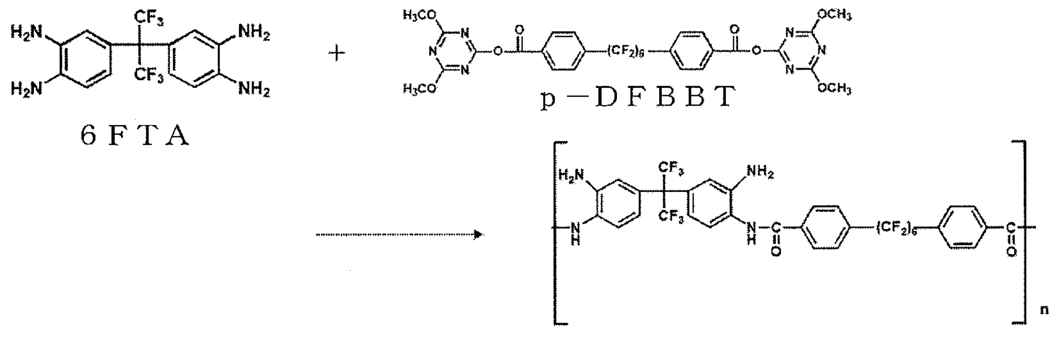

6FTA

p-DFBBT

PA (6FTA-p-DFBBT)

6FTA (0.364 g, 1.0 mmol) and 2 mL of NMP were charged into a 100 mL three-necked flask equipped with a stirrer and a nitrogen introduction tube, made to dissolve, and the resulting mixture was cooled to 0°C. Next, a triazine-based active diester (p-DFBBT) (0.821 g, 1.0 mmol) was added and dissolved, and the resulting mixture was reacted for 1 hour at 0°C. The temperature was increased in steps to 80°C, and the mixture was reacted for 24 hours at 80°C. After completion of the reaction, the polymerization solution was charged into distilled water (300 mL) to precipitate a fluorinated amide compound. The precipitated fluorinated amide compound was collected by suction filtration, and dried under reduced pressure at room temperature for 12 hours. The crude yield was 97%. The dried fluorinated amide compound was dissolved in NMP (5 mL) and purified by reprecipitation with distilled water to obtain a fluorinated amide compound (6FTA-p-DFBBT) in the form of a pale yellow powder (yield amount: 0.663 g, yield: 74%). It was confirmed by IR and $^1$H-NMR that a fluorinated amide compound was produced.

The physical properties of the obtained fluorinated amide compound are shown below.

FT-IR (KBr, $cm^{-1}$): 3366 (N-H), 1664 (C=O), 1519 (C=C)
$^1$H-NMR (DMSO-$d_6$, ppm): 5.38 (s, 4H, $NH_2$), 6.79 (d, 2H, Ar-H), 6.93 (d, 2H, Ar-H), 7.26 (s, 2H, Ar-H), 7.83 (d, 4H, Ar-H), 8.17 (d, 4H, Ar-H), 9.94 (s, 2H, NHCO)
Logarithmic viscosity ($\eta_{inh}$): 0.34 dL/g (NMP solution having a 0.5 g/dL concentration, measured at 30°C)
GPC: Number average molecular weight ($M_n$) 25,000, molecular weight distribution ($M_w/M_n$ 2.0
Solubility: Dissolved in NMP, DMAc, DMF, DMSO, THF, acetone, and methanol at room temperature

<Example 10>

Fluorinated amide compound (6FTAPh-p-DFBBT)

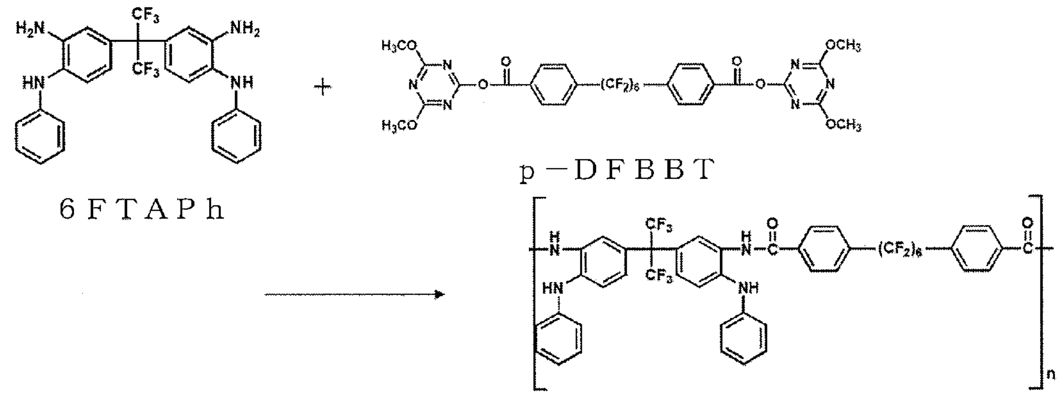

6FTAPh

p-DFBBT

PA (6FTAPh-p-DFBBT)

6FTAPh (0.516 g, 1.0 mmol) and NMP (2 mL) were charged into a 100 mL three-necked flask equipped with a stirrer and a nitrogen introduction tube and made to dissolve. Next, a triazine-based active diester (p-DFBBT) (0.821 g, 1.0 mmol) was added and dissolved. The temperature was increased in steps to 120°C, and the mixture was reacted for 24 hours at 120°C. After completion of the reaction, the polymerization solution was charged into distilled water (300 mL) to precipitate a fluorinated amide compound. The precipitated fluorinated amide compound was collected by suction filtration, and dried under reduced pressure at room temperature for 12 hours. The crude yield was 96%. The dried fluorinated amide compound was dissolved in NMP (5 mL) and purified by reprecipitation with distilled water to obtain a fluorinated amide compound (6FTAPh-p-DFBBT) in the form of a white powder (yield amount: 0.695 g, yield: 68%).

The physical properties of the obtained fluorinated amide compound are shown below.

FT-IR (KBr, $cm^{-1}$): 3341 (NHCO), 1672 (C=O), 1598 (C=C), 1532 (C=C)
$^1$H-NMR ($CDCl_3$, ppm): 6.36 (s, 2H, Ar-NH-Ar), 6.93 (m, 10H, Ar-H), 7.22 (s, 2H, Ar-H), 7.63 (t, 4H, Ar-H), 7.83-7.90 (m, 8H, Ar-H), 8.80 (s, 2H, NHCO) Logarithmic viscosity ($\eta_{inh}$): 0.37 dL/g (NMP solution having a 0.5 g/dL concentration, measured at 30°C)
GPC: Number average molecular weight ($M_n$) 59,000, molecular weight distribution ($M_w/M_n$) 1.3
Solubility: Dissolved in NMP, DMAc, DMF, DMSO, THF, chloroform, and acetone at room temperature

<Example 11>

Fluorinated nitrogen-containing heterocyclic ring-containing compound (imidazole compound (6FTA-p-DFBBT)

[0177]

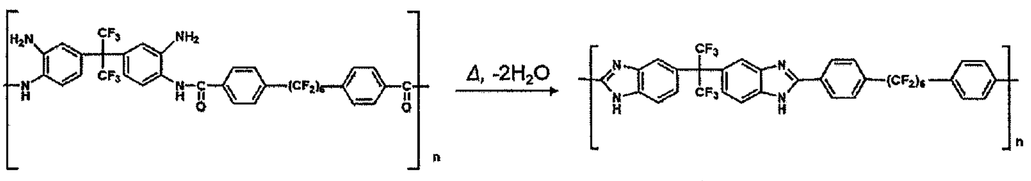


PBI (6FTA-p-DFBBT)

A powder of a dried fluorinated amide compound (6FTA-p-DFBBT) was thermally processed in a reduced-pressure dryer at for 6 hours at 60°C, 6 hours at 100°C, 1 hour at 150°C, 1 hour at 200°C, 1 hour at 250°C, 1 hour at 300°C, and 1 hour at 350°C to obtain a powder of a brown imidazole compound.

The physical properties of the obtained imidazole compound are shown below.

FT-IR (KBr, $cm^{-1}$): 1,551 (C=C), 1,451 (C=N), 723 (C-F)
Elemental analysis: Measured C, 49.72%; H, 2.33%; N, 6.60%
Calculated C, 50.37%; H, 1.93%; N, 6.71%
Solubility: Dissolved in NMP

Glass transition temperature: 240°C (DSC measurement)
5% Weight loss temperature: 429°C (in air), 442°C (in nitrogen) (TGA) 10% Weight loss temperature: 478°C (in air), 497°C (in nitrogen) (TGA) Carbonization yield: 57% (800°C in nitrogen) (TGA)
Cut-off wavelength: 342 nm
Transmission at 500 nm: 86%
Average refractive index ($n_{ave}$): 1.565 (d line)
Dielectric constant ($\varepsilon$) calculated from refractive index: 2.69 ($\varepsilon = 1.10 \times n_{ave}^2$)

<Example 12>

Fluorinated nitrogen-containing heterocyclic ring-containing compound (imidazole compound :6FTAPh-p-DFBBT)

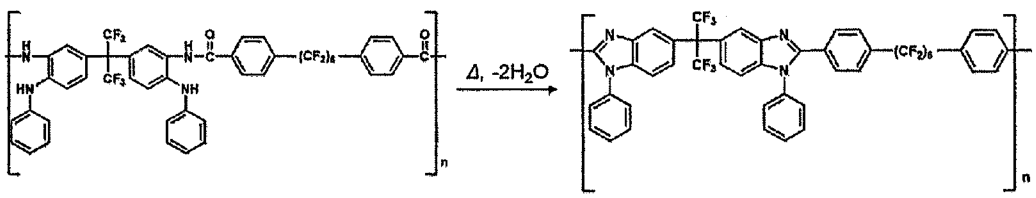


PBI (6FTAPh−p−DFBBT)

A powder of a dried fluorinated amide compound (6FTAPh-p-DFBBT) was thermally processed in a reduced-pressure dryer at for 6 hours at 60°C, 6 hours at 100°C, 1 hour at 150°C, 1 hour at 200°C, 1 hour at 250°C, 1 hour at 300°C, and 1 hour at 350°C to obtain a powder of a brown imidazole compound.
The physical properties of the obtained imidazole compound are shown below.

FT-IR (KBr, $cm^{-1}$): 1,596 (C=C), 1,503 (C=C), 1,455 (C=N)
Solubility: Dissolved in NMP, DMAc, DMF, and THF at room temperature Glass transition temperature: 211°C (DSC)
5% Weight loss temperature: 503°C (in air), 519°C (in nitrogen) (TGA)
10% Weight loss temperature: 532°C (in air), 542°C (in nitrogen) (TGA) Carbonization yield: 61% (800°C in nitrogen) (TGA)
Cut-off wavelength: 340 nm
Transmission at 500 nm: 77%
Average refractive index ($n_{ave}$): 1.545 (d line)
Dielectric constant ($\varepsilon$) calculated from refractive index: 2.63 ($\varepsilon = 1.10 \times n_{ave}^2$)

<Synthesis example 3>

1,6-Bis(3-aminophenyl)dodecafluorohexane (mAPDF6)

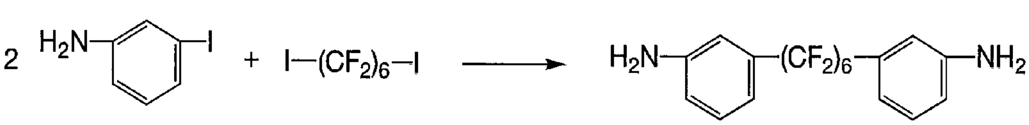

mAPDF6

3-iodoaniline (5.0 g, 22.8 mmol) and DMSO (20 mL) were charged into a 100 mL eggplant flask equipped with a stirrer, a Dimroth condenser, and a three-way cock, and made to dissolve. Then, the mixture was quickly charged with copper powder (7.2 g, 114 mmol) so as not to oxidize in air, and quickly charged with 1,6-diiodoperfluorohexane (6.3 g, 11.4 mmol) before it deliquesced. Then, the temperature was raised to 120°C, and the mixture was reacted while stirring for 24 hours at 120°C. After completion of the reaction, the mixture was cooled to room temperature and suction filtered to recover the DMSO solution. The DMSO was distilled off from the DMSO solution by vacuum distillation to obtain a crude product. The crude product was dissolved in dichloromethane, washed with distilled water, the dichloromethane solution was recovered, and the resulting product was dried over anhydrous sodium sulfate overnight. The dichloromethane was distilled off with an evaporator to obtain a yellow crude product. The yellow crude product was recrystallized from a mixed solvent of methanol / distilled water, and dried under reduced pressure at 60°C. The product was dissolved in methanol and decolorized with activated carbon. After removing the methanol, the product was dried under reduced pressure at 60°C overnight to obtain mAPDF6 (yield amount: 3.0 g, yield: 54%) in the form of a yellow powder.

The structure was confirmed by $^{1}$H-NMR, $^{13}$C-NMR, $^{19}$F-NMR, and elemental analysis.

$^{1}$H-NMR ($CDCl_3$, ppm) : 3.81 (s, 4H, $NH_2$), 6.83 (d, 2H, Ar-H), 6.85 (s, 2H, Ar-H), 6. 95 (d, 2H, Ar-H), 7.24 (t, 2H, Ar-H)
$^{13}$C-NMR ($CDCl_3$, ppm): 113.1, 116.9, 118.2, 129.6, 130.3, 146.7
$^{19}$F-NMR ($CDCl_3$, ppm): -121.9, -121.3, -110.7
Melting point: 79.4°C (DSC)
Elemental analysis: Calculated C, 44.64%; H, 2.50%; N, 5.78%
Measured C, 44.75%; H, 2.80%; N, 5.52%

<Example 13>

1,6-Bis(4-chlorocarbonylphenyl)dodecafluorohexane (CCPDF6)

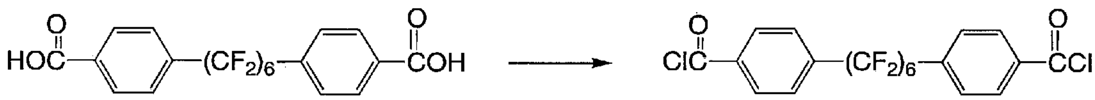

CPDF6 CCPDF6

CPDF6 (5.38 g, 9.9 mmol) and thionyl chloride (40 mL) were charged into an eggplant flask (100 mL) equipped with a stirrer, a condenser, and a calcium chloride tube, the temperature was slowly raised to 85°C, and the mixture was stirred for 1 hour. The mixture was allowed to cool to room temperature, and excess thionyl chloride was distilled off by vacuum distillation to obtain a solid product. The obtained solid product was purified by sublimation (140°C/0.8 Torr) to obtain a white needle-like crystal product (yield amount: 4.4 g, yield: 77%) .

The structure was confirmed by FT-IR, $^{1}$H-NMR, $^{13}$C-NMR, $^{19}$F-NMR, and elemental analysis.

FT-IR (KBr, $cm^{-1}$): 3063 (Ar-H), 1746 (C=O), 1142 (C-F)
$^{1}$H-NMR ($CDCl_3$, ppm): 8.24 (d, 4H, Ar-H), 7.75 (d, 4H, Ar-H)
$^{13}$C-NMR ($CDCl_3$, ppm): 127.78, 131.40, 135.49, 136.44, 167.76,
$^{19}$F-NMR ($CDCl_3$, ppm): -111.33, -121.10, -121.60
Melting point: 129 to 130°C
Elemental analysis: Calculated C, 41.48%; H, 1.39%
Measured C, 41.43%; H, 1.48%

<Example 14>

Fluorinated amide compound and fluorinated nitrogen-containing heterocyclic ring-containing compound (imide compound: APDF6/BisAAF (50/50)-6FDA)

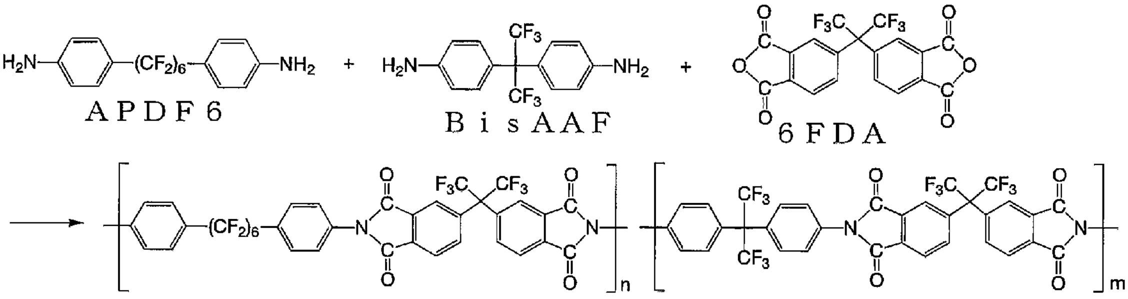

APDF6 BisAAF 6FDA

PI (APDF6/BisAAF-6FDA)

A fluorinated amide compound was synthesized by carrying out an experiment in the same manner as the above-mentioned examples using APDF6 (0.363 g, 0.75 mmol), 2,2'-bis(4-aminophenyl)hexafluoropropane (BisAAF) (0.251 g, 0.75 mmol), and 4,4'-(hexafluoroisopropylidene)diphthalic acid dianhydride (6FDA) (0.666 g, 1.50 mmol) . The logarithmic viscosity ($\eta_{inh}$) of the fluorinated amide compound was 0.50 dL/g (NMP solution having a concentration of 0.5 g/dL, measured at 30°C). The fluorinated amide compound was then heated in the same manner to obtain a pale yellow transparent fluorinated nitrogen-containing heterocyclic ring-containing compound (imide compound) film (thickness 64 $\mu$m).

The physical properties of the obtained fluorinated nitrogen-containing heterocyclic ring-containing compound (imide compound) film are shown below.

Number average molecular weight (Mn): 31,000, molecular weight distribution (Mw/Mn): 2.5 (GPC)
Solubility: Dissolved in NMP, DMAc, cyclohexanone, THF, chloroform, ethyl acetate, and acetone at room temperature
5% Weight loss temperature: 502°C (in air), 524°C (in nitrogen) (TGA) 10% Weight loss temperature: 522°C (in air), 542°C (in nitrogen) (TGA) Carbonization yield: 52% (800°C in nitrogen) (TGA)
Glass transition temperature: 265°C (DSC), 258°C (DMA), 267°C (TMA) Thermal expansion coefficient (CTE): 62 (ppm/°C)
Cut-off wavelength: 354 nm
Transmission at 500 nm: 84%
Average refractive index ($n_{ave}$): 1.537 (d line)
Dielectric constant ($\varepsilon$) calculated from refractive index: 2.60 ($\varepsilon = 1.10 \times n_{ave}^2$)
Dielectric constant (Dk) at 10 GHz: 2.49
Dielectric tangent (Df) at 10 GHz: 0.0043

<Example 15>

Fluorinated amide compound and fluorinated nitrogen-containing heterocyclic ring-containing compound (imide compound: APDF6/TFMB (50/50)-6FDA)

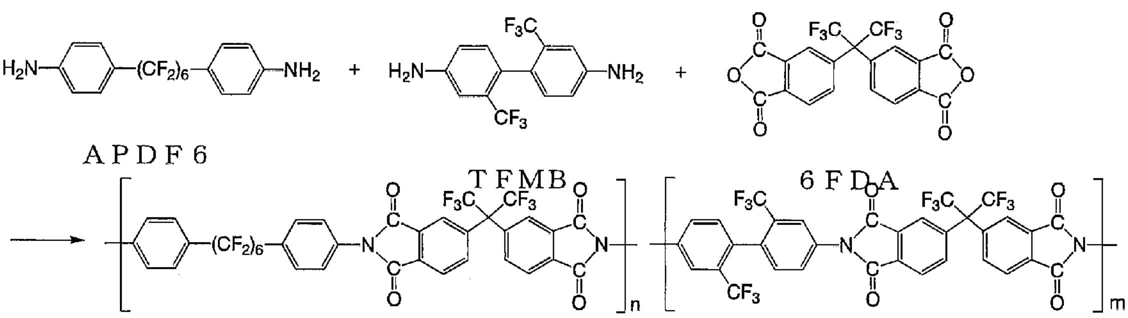

APDF6 TFMB 6FDA

PI (APDF6/TFMB-6FDA)

A fluorinated amide compound was synthesized by carrying out an experiment in the same manner as the above-mentioned examples using APDF6 (0.363 g, 0.75 mmol), 2,2'-bis(trifluoromethyl)benzidine (TFMB) (0.240 g, 0.75 mmol), and 4,4'-(hexafluoroisopropylidene)diphthalic acid dianhydride (6FDA) (0.666 g, 1.50 mmol). The logarithmic viscosity ($\eta_{inh}$) of the fluorinated amide compound was 0.37 dL/g (NMP solution having a concentration of 0.5 g/dL, measured at 30°C). The fluorinated amide compound was then heated in the same manner to obtain a pale yellow transparent fluorinated nitrogen-containing heterocyclic ring-containing compound (imide compound) film (thickness 69 μm).

The physical properties of the obtained fluorinated nitrogen-containing heterocyclic ring-containing compound (imide compound) film are shown below.

Number average molecular weight (Mn): 50,000, molecular weight distribution (Mw/Mn): 1.8 (GPC)
Solubility: Dissolved in NMP, DMAc, cyclohexanone, THF, chloroform, ethyl acetate, and acetone at room temperature
5% Weight loss temperature: 505°C (in air), 538°C (in nitrogen) (TGA)
10% Weight loss temperature: 525°C (in air), 560°C (in nitrogen) (TGA) Carbonization yield: 52% (800°C in nitrogen) (TGA)
Glass transition temperature: 268°C (DSC), 257°C (DMA), 261°C (TMA) Thermal expansion coefficient (CTE): 43 (ppm/°C)
Cut-off wavelength: 355 nm
Transmission at 500 nm: 83%
Average refractive index ($n_{ave}$): 1.538 (d line)
Dielectric constant ($\varepsilon$) calculated from refractive index: 2.60 ($\varepsilon = 1.10 \times n_{ave}^2$)
Dielectric constant (Dk) at 10 GHz: 2.52
Dielectric tangent (Df) at 10 GHz: 0.0039

<Example 16>

Fluorinated amide compound and fluorinated nitrogen-containing heterocyclic ring-containing compound (imide compound: APHF8/BisAAF (50/50)-6FDA)

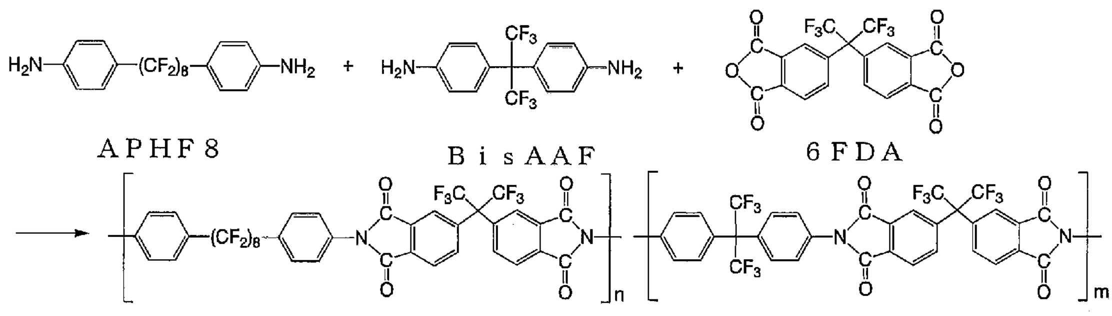
APHF8 BisAAF 6FDA

PI (APHF8/BisAAF−6FDA)

A fluorinated amide compound was synthesized by carrying out an experiment in the same manner as the above-mentioned examples using APHF8 (0.443 g, 0.75 mmol), BisAAF (0.251 g, 0.75 mmol), and 4,4'-(hexafluoroisopropylidene)diphthalic acid dianhydride (6FDA) (0.666 g, 1.50 mmol) . The logarithmic viscosity ($\eta_{inh}$) of the fluorinated amide compound was 0.32 dL/g (NMP solution having a concentration of 0.5 g/dL, measured at 30°C). The fluorinated amide compound was then heated in the same manner to obtain a pale yellow transparent fluorinated nitrogen-containing heterocyclic ring-containing compound (imide compound) film (thickness 47 μm).

The physical properties of the obtained fluorinated nitrogen-containing heterocyclic ring-containing compound (imide compound) film are shown below.

Number average molecular weight (Mn): 30,000, molecular weight distribution (Mw/Mn): 2.4 (GPC)
Solubility: Dissolved in NMP, DMAc, THF, chloroform, ethyl acetate, and acetone at room temperature
5% Weight loss temperature: 509°C (in air), 520°C (in nitrogen) (TGA) 10% Weight loss temperature: 529°C (in air),

539°C (in nitrogen) (TGA) Carbonization yield: 51% (800°C in nitrogen) (TGA)
Glass transition temperature: 252°C (DSC), 247°C (DMA), 246°C (TMA) Thermal expansion coefficient (CTE): 75 (ppm/°C)
Cut-off wavelength: 352 nm
Transmission at 500 nm: 73%
Average refractive index ($n_{ave}$): 1.528 (d line)
Dielectric constant ($\varepsilon$) calculated from refractive index: 2.57 ($\varepsilon = 1.10 \times n_{ave}^2$)
Dielectric constant (Dk) at 10 GHz: 2.49
Dielectric tangent (Df) at 10 GHz: 0.0023

<Example 17>

Fluorinated amide compound and fluorinated nitrogen-containing heterocyclic ring-containing compound (imide compound: APHF8/TFMB (50/50)-6FDA)

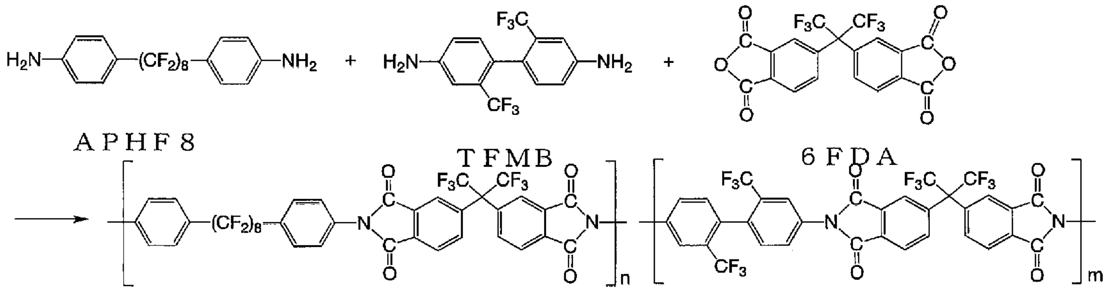
APHF8
TFMB
6FDA

PI (APHF8/TFMB-6FDA)

A fluorinated amide compound was synthesized by carrying out an experiment in the same manner as the above-mentioned examples using APHF8 (0.443 g, 0.75 mmol), TFMB (0.240 g, 0.75 mmol), and 4,4'-(hexafluoroisopropylidene)diphthalic acid dianhydride (6FDA) (0.666 g, 1.50 mmol) . The logarithmic viscosity ($\eta_{inh}$) of the fluorinated amide compound was 0.42 dL/g (NMP solution having a concentration of 0.5 g/dL, measured at 30°C). The fluorinated amide compound was then heated in the same manner to obtain a pale yellow transparent fluorinated nitrogen-containing heterocyclic ring-containing compound (imide compound) film (thickness 86 $\mu$m).

The physical properties of the obtained fluorinated nitrogen-containing heterocyclic ring-containing compound (imide compound) film are shown below.

Number average molecular weight (Mn): 55,000, molecular weight distribution (Mw/Mn): 2.4 (GPC)
Solubility: Dissolved in NMP, DMAc, cyclohexanone, THF, chloroform, ethyl acetate, and acetone at room temperature
5% Weight loss temperature: 525°C (in air), 529°C (in nitrogen) (TGA) 10% Weight loss temperature: 545°C (in air), 550°C (in nitrogen) (TGA) Carbonization yield: 51% (800°C in nitrogen) (TGA)
Glass transition temperature: 255°C (DSC), 241°C (DMA), 239°C (TMA) Thermal expansion coefficient (CTE): (55 ppm/°C)
Cut-off wavelength: 350 nm
Transmission at 500 nm: 80%
Average refractive index ($n_{ave}$): 1.528 (d line)
Dielectric constant ($\varepsilon$) calculated from refractive index: 2.57 ($\varepsilon = 1.10 \times n_{ave}^2$)
Dielectric constant (Dk) at 10 GHz: 2.57
Dielectric tangent (Df) at 10 GHz: 0.0031

<Comparative Example 1>

Fluorinated amide compound and fluorinated nitrogen-containing heterocyclic ring-containing compound (imide compound: BisAAF-6FDA)

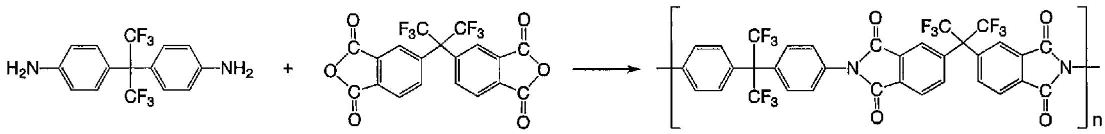

BisAAF 6FDA PI (BisAAF-6FDA)

A 100 mL three-necked flask equipped with a stirring rod, a stirring blade, a Dimroth condenser, and a nitrogen introduction tube was heated and dried with a burner, then BisAAF (0.502 g, 1.50 mmol) and distilled NMP (3 mL) were added, and the mixture was stirred to dissolve. Then, 4,4'-(hexafluoroisopropylidene)diphthalic acid dianhydride (6FDA) (0.666 g, 1.50 mmol) was added, the temperature was gradually raised, and the mixture was reacted by stirring for 18 hours at 60°C to obtain a solution of the fluorinated amide compound. The logarithmic viscosity ($\eta_{inh}$) of the fluorinated amide compound was 0.58 dL/g (NMP solution having a concentration of 0.5 g/dL, measured at 30°C). The fluorinated amide compound was soluble in NMP, DMAc, γ-butyrolactone, acetone, methanol, ethanol, ethyl acetate, THF, and cyclohexanone. The solution was cooled to room temperature, distilled NMP (2 mL) was added, the mixture was stirred to dilute the concentration of the entire solution, and the solution was cast on a glass plate. The cast mixture was dried under reduced pressure for 3 hours at room temperature, then dried under reduced pressure in a program consisting of 6 hours at 60°C, 1 hour at 100°C, 1 hour at 150°C, 1 hour at 200°C, 1 hour at 250°C, and 1 hour at 300°C to obtain a pale yellow transparent fluorinated nitrogen-containing heterocyclic ring-containing compound (imide compound) film (thickness 58 μm).

The physical properties of the obtained fluorinated nitrogen-containing heterocyclic ring-containing compound (imide compound) film are shown below.

Number average molecular weight (Mn) 26,000, molecular weight distribution (Mw/Mn) 2.3 (GPC)
Solubility: Dissolved in DMF, DMAc, THF, chloroform, ethyl acetate, and acetone at room temperature
5% Weight loss temperature: 508°C (in air), 524°C (in nitrogen) (TGA) 10% Weight loss temperature: 525°C (in air), 539°C (in nitrogen) (TGA) Carbonization yield: 54% (800°C in nitrogen) (TGA)
Glass transition temperature: 311°C (DSC), 313°C (DMA), 310°C (TMA) Thermal expansion coefficient (CTE): 69 ppm/°C
Cut-off wavelength: 354 nm
Transmission at 500 nm: 88%
Average refractive index ($n_{ave}$): 1.550 (d line)
Dielectric constant (ε) calculated from refractive index: 2.64 ($\varepsilon = 1.10 \times n_{ave}^2$)
Dielectric constant (Dk) at 10 GHz: 2.65
Dielectric tangent (Df) at 10 GHz: 0.0070

<Comparative Example 2>

Fluorinated amide compound and fluorinated nitrogen-containing heterocyclic ring-containing compound (imide compound: TFMB-6FDA)

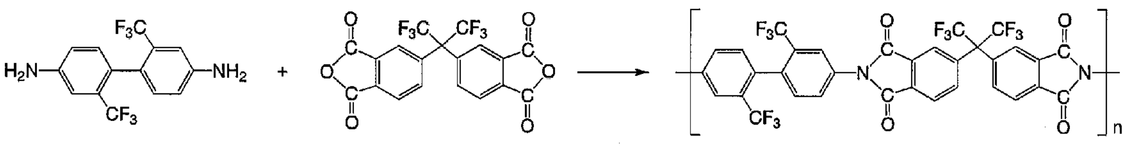

TFMB 6FDA PI (TFMB-6FDA)

A 100 mL three-necked flask equipped with a stirring rod, a stirring blade, a Dimroth condenser, and a nitrogen

introduction tube was heated and dried with a burner, then TFMB (0.480 g, 1.50 mmol) and distilled NMP (3 mL) were added, and the mixture was stirred to dissolve. Then, 4,4'-(hexafluoroisopropylidene)diphthalic acid dianhydride (6FDA) (0.666 g, 1.50 mmol) was added, the temperature was gradually raised, and the mixture was reacted by stirring for 18 hours at 60°C to obtain a solution of the fluorinated amide compound. The logarithmic viscosity ($\eta_{inh}$) of the fluorinated amide compound was 0.58 dL/g (NMP solution having a concentration of 0.5 g/dL, measured at 30°C). The fluorinated amide compound was soluble in NMP, DMAc, γ-butyrolactone, acetone, methanol, ethanol, ethyl acetate, THF, and cyclohexanone. The solution was cooled to room temperature, distilled NMP (2 mL) was added, the mixture was stirred to dilute the concentration of the entire solution, and the solution was cast on a glass plate. The cast mixture was dried under reduced pressure for 3 hours at room temperature, then dried under reduced pressure in a program consisting of 6 hours at 60°C, 1 hour at 100°C, 1 hour at 150°C, 1 hour at 200°C, 1 hour at 250°C, and 1 hour at 300°C to obtain a pale yellow transparent fluorinated nitrogen-containing heterocyclic ring-containing compound (imide compound) film (thickness 66 μm).

The physical properties of the obtained fluorinated nitrogen-containing heterocyclic ring-containing compound (imide compound) film are shown below.

Number average molecular weight (Mn) 21,000, molecular weight distribution (Mw/Mn) 1.9 (GPC)
Solubility: Dissolved in DMF, DMAc, cyclohexanone, THF, ethyl acetate, and acetone at room temperature
5% Weight loss temperature: 527°C (in air), 530°C (in nitrogen) (TGA) 10% Weight loss temperature: 543°C (in air), 552°C (in nitrogen) (TGA) Carbonization yield: 52% (800°C in nitrogen) (TGA)
Glass transition temperature: 332°C (DSC), 331°C (DMA), 323°C (TMA) Thermal expansion coefficient (CTE): 68 ppm/°C
Cut-off wavelength: 351 nm
Transmission at 500 nm: 84%
Average refractive index ($n_{ave}$): 1.554 (d line)
Dielectric constant (ε) calculated from refractive index: 2.66 ($\varepsilon = 1.10 \times n_{ave}^2$)
Dielectric constant (Dk) at 10 GHz: 2.74
Dielectric tangent (Df) at 10 GHz: 0.0066

## Claims

1. A fluorinated amide compound comprising a repeating unit represented by Formula (1):

Formula (1):

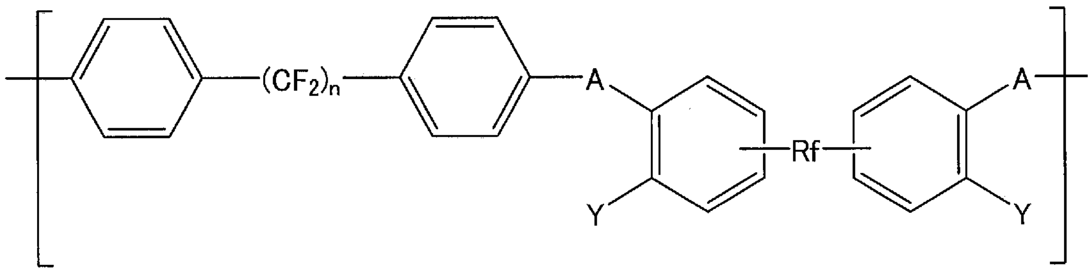

wherein

n represents an integer of 4 to 8,
Rf represents a single bond, $-SO_2-$, -O-, -CO-, a divalent non-fluorinated organic group, or a divalent fluorinated organic group,
A represents an amide bond,
each Y independently represents $-COR^1$ or $-NHR^2$,
each $R^1$ independently represents OH, an optionally-substituted linear or branched chain alkoxy group, an optionally-substituted aromatic oxy group, or a halogen atom, and
each $R^2$ independently represents H or a monovalent organic group.

2. The fluorinated amide compound according to claim 1, wherein an average degree of polymerization of the repeating unit represented by Formula (1) is 2 to 100.

3. The fluorinated amide compound according to claim 1 or 2, wherein the repeating unit represented by Formula (1) is a repeating unit represented by Formula (1a):

Formula (1a):

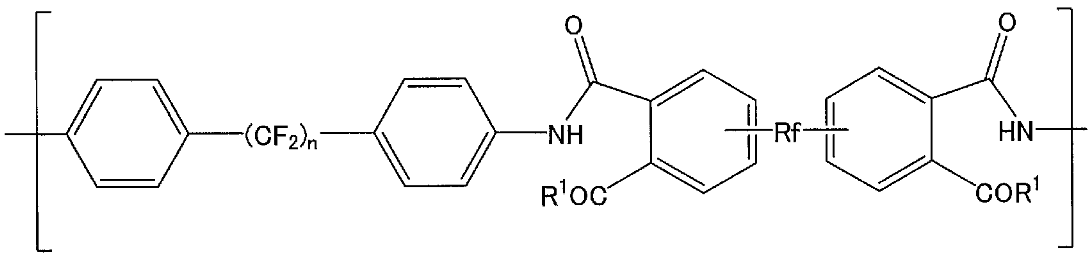

wherein

n represents an integer of 4 to 8,
Rf represents a single bond, $-SO_2-$, -O-, -CO-, a divalent non-fluorinated organic group, or a divalent fluorinated organic group, and
each $R^1$ independently represents OH, an optionally-substituted linear or branched chain alkoxy group, an optionally-substituted aromatic oxy group, or a halogen atom.

4. The fluorinated amide compound according to claim 1 or 2, wherein the repeating unit represented by Formula (1) is a repeating unit represented by Formula (1b):

Formula (1b):

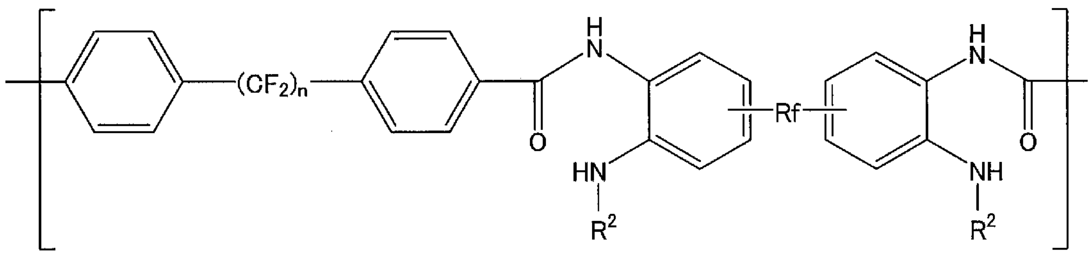

wherein

n represents an integer of 4 to 8,
Rf represents a single bond, $-SO_2-$, -O-, -CO-, a divalent non-fluorinated organic group, or a divalent fluorinated organic group, and
each $R^2$ independently represents H or a monovalent organic group.

5. A fluorinated nitrogen-containing heterocyclic ring-containing compound comprising a repeating unit represented by Formula (3),

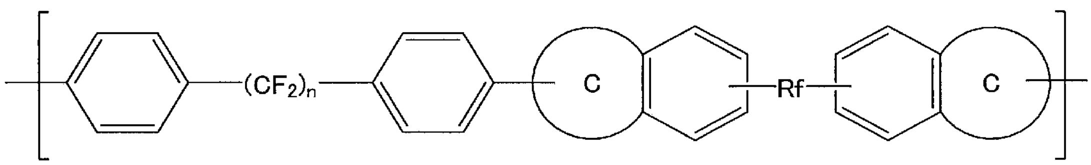

wherein

n represents an integer of 4 to 8,

Rf represents a single bond, $-SO_2-$, -O-, -CO-, a divalent non-fluorinated organic group, or a divalent fluorinated organic group, and
a ring C represents an optionally-substituted imide ring or benzimidazole ring.

**6.** The fluorinated nitrogen-containing heterocyclic ring-containing compound according to claim 5, wherein an average degree of polymerization of the repeating unit represented by Formula (3) is 2 to 100.

**7.** The fluorinated nitrogen-containing heterocyclic ring-containing compound according to claim 5 or 6, wherein the repeating unit represented by Formula (3) is a repeating unit represented by Formula (3a),

Formula (3a):

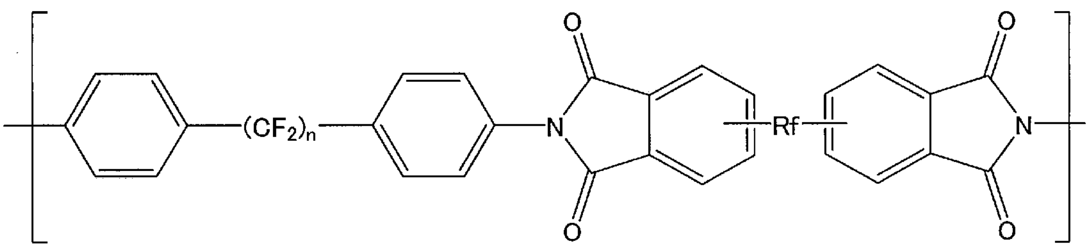

wherein

n represents an integer of 4 to 8, and
Rf represents a single bond, $-SO_2-$, -O-, -CO-, a divalent non-fluorinated organic group, or a divalent fluorinated organic group.

**8.** The fluorinated nitrogen-containing heterocyclic ring-containing compound according to claim 5 or 6, wherein the repeating unit represented by Formula (3) is a repeating unit represented by Formula (3b),

Formula (3b):

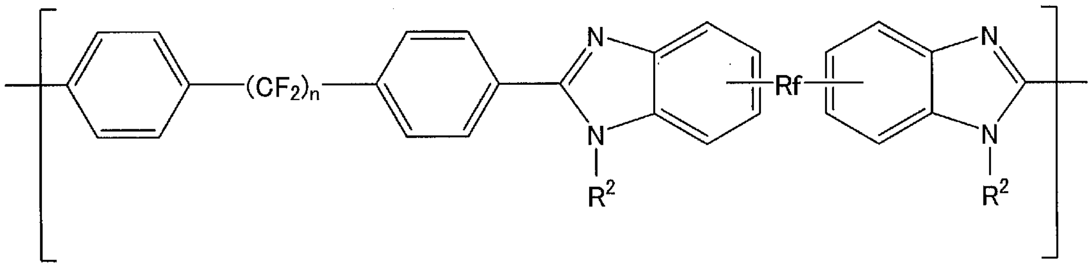

wherein

n represents an integer of 4 to 8,
Rf represents a single bond, $-SO_2-$, -O-, -CO-, a divalent non-fluorinated organic group, or a divalent fluorinated organic group, and
each $R^2$ independently represents H or a monovalent organic group.

**9.** A low dielectric material comprising the fluorinated amide compound according to any of claims 1 to 4 or the fluorinated nitrogen-containing heterocyclic ring-containing compound according to any of claims 5 to 8.

**10.** A printed circuit board comprising the fluorinated amide compound according to any of claims 1 to 4 or the fluorinated nitrogen-containing heterocyclic ring-containing compound according to any of claims 5 to 8.

**11.** A fluorinated compound (4a-1) represented by Formula (4a-1):

Formula (4a-1):

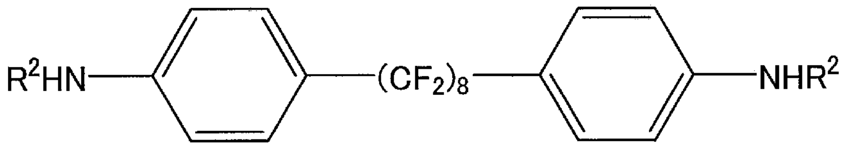

wherein each $R^2$ independently represents H or a monovalent organic group.

**12.** A fluorinated compound (4B) represented by Formula (4B):

Formula (4B):

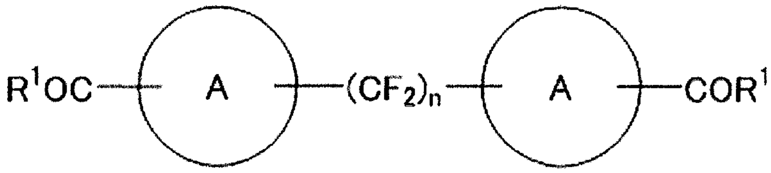

wherein

n represents an integer of 4 to 8,
ring A represents a hydrocarbon ring, and
each $R^1$ independently represents OH, an optionally-substituted linear or branched chain alkoxy group, an optionally-substituted aromatic oxy group, or a halogen atom.

**INTERNATIONAL SEARCH REPORT**

International application No.
**PCT/JP2021/017952**

**A. CLASSIFICATION OF SUBJECT MATTER**

***C07C 63/72***(2006.01)i; ***C07C 211/52***(2006.01)i; ***C08G 73/10***(2006.01)i; ***C08G 73/18***(2006.01)i
FI: C08G73/10; C08G73/18; C07C63/72; C07C211/52 CSP

According to International Patent Classification (IPC) or to both national classification and IPC

**B. FIELDS SEARCHED**

Minimum documentation searched (classification system followed by classification symbols)

C07C63/72; C07C211/52; C08G73/10; C08G73/18

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 04-288344 A (SANYO CHEMICAL INDUSTRIES, LTD.) 13 October 1992 (1992-10-13) claims, paragraphs [0031]-[0035], examples | 1-3, 5-7, 9 |
| X | YAKUBOVICHI, A. YA. et al. Vysokomolukulyarnye Soedineniya. Seriya A., 1970, vol. 12, no. 11, pp. 2520-2531, particularly, pp. 2520, 2526, 2527 pp. 2520, 2526, 2527 | 1-9, 12 |
| Y | pp. 2520, 2526, 2527 | 10 |
| Y | WO 2019/54471 A1 (DAIKIN INDUSTRIES, LTD.) 21 March 2019 (2019-03-21) claims | 10 |
| Y | JP 2018-145303 A (KANEKA CORP.) 20 September 2018 (2018-09-20) claims | 10 |
| X | JP 6374867 B2 (NICHIAS CORP.) 15 August 2018 (2018-08-15) claims, paragraph [0036], examples | 11 |
| X | EP 3031848 A1 (ABB TECHNOLOGY LTD.) 15 June 2016 (2016-06-15) claims, fig. 1 | 11 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 June 2021** | **06 July 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2021/017952**

| Patent document cited in search report | Publication date (day/month/year) | Patent family member(s) | Publication date (day/month/year) |
|---|---|---|---|
| JP 04-288344 A | 13 October 1992 | (Family: none) | |
| WO 2019/54471 A1 | 21 March 2019 | EP 3666814 A1<br>claims<br>KR 10-2020-0047701 A<br>CN 111108145 A<br>TW 201920376 A | |
| JP 2018-145303 A | 20 September 2018 | (Family: none) | |
| JP 6374867 B2 | 15 August 2018 | US 2016/0185892 A1<br>claims, paragraph [0050], examples<br>WO 2015/019581 A1<br>EP 3031851 A1<br>CN 105452361 A<br>KR 10-2016-0040541 A | |
| EP 3031848 A1 | 15 June 2016 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9208699 A


### Non-patent literature cited in the description


- Latest Polyimides -Basics and Applications. NTS Co., Ltd, 28 January 2002, 292-326